# EUROPEAN PATENT SPECIFICATION

(11) **EP 3 482 329 B1**
(45) Date of publication and mention of the grant of the patent: **07.10.2020**
(21) Application number: 17714441.7
(22) Date of filing: 30.03.2017
(51) Int. Cl.: G16B 30/10

(54) **A COMPUTER-IMPLEMENTED AND REFERENCE-FREE METHOD FOR IDENTIFYING VARIANTS IN NUCLEIC ACID SEQUENCES**
COMPUTERIMPLEMENTIERTES UND BEZUGSFREIES VERFAHREN ZUR IDENTIFIZIERUNG VON VARIANTEN IN NUKLEINSÄURESEQUENZEN
PROCÉDÉ SANS RÉFÉRENCE ET MIS EN OEUVRE PAR ORDINATEUR POUR L'IDENTIFICATION DE VARIANTS DANS DES SÉQUENCES D'ACIDE NUCLÉIQUE

(30) Priority: 08.07.2016 EP 16178577
(43) Date of publication of application: 15.05.2019
(73) Proprietor: Barcelona Supercomputing Center-Centro Nacional de Supercomputación, 08034 Barcelona (ES); Institució Catalana de Recerca i Estudis Avançats, 08010 Barcelona (ES); Universitat Politècnica de Catalunya, 08034 Barcelona (ES)
(72) Inventor: CARRERA PEREZ, David, 08034 Barcelona (ES); POLO, Jordà, 08034 Barcelona (ES); CADENELLI, Nicola, 08034 Barcelona (ES); TORRENTS ARENALES, David, 08034 Barcelona (ES); PLANAS, Mercè, 08034 Barcelona (ES)
(74) Representative: ZBM Patents - Zea, Barlocci & Markvardsen
(86) International application number: PCT/EP2017/057504
(87) International publication number: WO 2018/007034

(56) References cited:
- WO-A1-2015/112619
- WO-A1-2016/062713
- KARL J V NORDSTRÖM ET AL: "Mutation identification by direct comparison of whole-genome sequencing data from mutant and wild-type individuals using k-mers", NATURE BIOTECHNOLOGY, vol. 31, no. 4, 10 March 2013 (2013-03-10) , pages 325-330, XP055183348, ISSN: 1087-0156, DOI: 10.1038/nbt.2515

### SEQUENCE LISTING

<110> Barcelona Supercomputing Center - Centro Nacional de Supercomputacion
<120> A computer-implemented and reference-free method for identifying
   variants
<130> P3565PC00
<150> EP16178577.9
   <151> 2016-07-08
<160> 147
<170> BiSSAP 1.0
<210> 1
   <211> 30
   <212> DNA
   <213> artificial sequences
<220>
   <221> source
   <222> 1..30
   <223> /mol_type="DNA" /note="Example sequence. Made up for explanation purposes" /organism="artificial sequences"
<400> 1
   cacggcagct gagtcaacag gttcttccca 30
<210> 2
   <211> 29
   <212> DNA
   <213> artificial sequences
<220>
   <221> source
   <222> 1..29
   <223> /mol_type="DNA" /note= "example" /organism="artificial sequences"
<400> 2
   cacggcagct gagtcaacag gttcttccc 29
<210> 3
   <211> 28
   <212> DNA
   <213> artificial sequences
<220>
   <221> source
   <222> 1..28
   <223> /mol_type="DNA" /note="example - omission of prefix and suffix of length 1" /organism="artificial sequences"
<400> 3
   acggcagctg agtcaacagg ttcttccc 28
<210> 4
   <211> 28
   <212> DNA
   <213> artificial sequences
<220>
   <221> source
   <222> 1..28
   <223> /mol_type="DNA" /note="omission of infix of length 2" /organism="artificial sequences"
<400> 4
   cacgagctga gtcaacaggt tcttccca 28
<210> 5
   <211> 29
   <212> DNA
   <213> artificial sequences
<220>
   <221> source
   <222> 1..29
   <223> /mol_type="DNA" /note="stem example" /organism="artificial sequences"
<400> 5
   cacggcagct gagtcaacag gttcttccc 29
<210> 6
   <211> 30
   <212> DNA
   <213> artificial sequences
<220>
   <221> source
   <222> 1..30
   <223> /mol_type="DNA" /note="stem example" /organism="artificial sequences"
<400> 6
   cacggcagct gagtcaacag gttcttccca 30
<210> 7
   <211> 30
   <212> DNA
   <213> artificial sequences
<220>
   <221> source
   <222> 1..30
   <223> /mol_type="DNA" /note="stem example" /organism="artificial sequences"
<400> 7
   cacggcagct gagtcaacag gttcttcccc 30
<210> 8
   <211> 30
   <212> DNA
   <213> artificial sequences
<220>
   <221> source
   <222> 1..30
   <223> /mol_type="DNA" /note="stem example" /organism="artificial sequences"
<400> 8
   cacggcagct gagtcaacag gttcttccct 30
<210> 9
   <211> 30
   <212> DNA
   <213> artificial sequences
<220>
   <221> source
   <222> 1..30
   <223> /mol_type="DNA" /note="stem example" /organism="artificial sequences"
<400> 9
   cacggcagct gagtcaacag gttcttcccg 30
<210> 10
   <211> 28
   <212> DNA
   <213> artificial sequences
<220>
   <221> source
   <222> 1..28
   <223> /mol_type="DNA" /note="example stem for inflections" /organism="artificial sequences"
<400> 10
   acggcagctg agtcaacagg ttcttccc 28
<210> 11
   <211> 30
   <212> DNA
   <213> artificial sequences
<220>
   <221> source
   <222> 1..30
   <223> /mol_type="DNA" /note="stem example" /organism="artificial sequences"
<400> 11
   aacggcagct gagtcaacag gttcttccca 30
<210> 12
   <211> 30
   <212> DNA
   <213> artificial sequences
<220>
   <221> source
   <222> 1..30
   <223> /mol_type="DNA" /note="stem example" /organism="artificial sequences"
<400> 12
   aacggcagct gagtcaacag gttcttcccc 30
<210> 13
   <211> 30
   <212> DNA
   <213> artificial sequences
<220>
   <221> source
   <222> 1..30
   <223> /mol_type="DNA" /note="stem example" /organism="artificial sequences"
<400> 13
   aacggcagct gagtcaacag gttcttccct 30
<210> 14
   <211> 30
   <212> DNA
   <213> artificial sequences
<220>
   <221> source
   <222> 1..30
   <223> /mol_type="DNA" /note="stem example" /organism="artificial sequences"
<400> 14
   aacggcagct gagtcaacag gttcttcccg 30
<210> 15
   <211> 30
   <212> DNA
   <213> artificial sequences
<220>
   <221> source
   <222> 1..30
   <223> /mol_type="DNA" /note="stem example" /organism="artificial sequences"
<400> 15
   cacggcagct gagtcaacag gttcttccca 30
<210> 16
   <211> 30
   <212> DNA
   <213> artificial sequences
<220>
   <221> source
   <222> 1..30
   <223> /mol_type="DNA" /note="stem example" /organism="artificial sequences"
<400> 16
   cacggcagct gagtcaacag gttcttcccc 30
<210> 17
   <211> 30
   <212> DNA
   <213> artificial sequences
<220>
   <221> source
   <222> 1..30
   <223> /mol_type="DNA" /note="stem example" /organism="artificial sequences"
<400> 17
   cacggcagct gagtcaacag gttcttccct 30
<210> 18
   <211> 30
   <212> DNA
   <213> artificial sequences
<220>
   <221> source
   <222> 1..30
   <223> /mol_type="DNA" /note="stem example" /organism="artificial sequences"
<400> 18
   cacggcagct gagtcaacag gttcttcccg 30
<210> 19
   <211> 30
   <212> DNA
   <213> artificial sequences
<220>
   <221> source
   <222> 1..30
   <223> /mol_type="DNA" /note="stem example" /organism="artificial sequences"
<400> 19
   tacggcagct gagtcaacag gttcttccca 30
<210> 20
   <211> 30
   <212> DNA
   <213> artificial sequences
<220>
   <221> source
   <222> 1..30
   <223> /mol_type="DNA" /note="stem example" /organism="artificial sequences"
<400> 20
   tacggcagct gagtcaacag gttcttcccc 30
<210> 21
   <211> 30
   <212> DNA
   <213> artificial sequences
<220>
   <221> source
   <222> 1..30
   <223> /mol_type="DNA" /note="stem example" /organism="artificial sequences"
<400> 21
   tacggcagct gagtcaacag gttcttccct 30
<210> 22
   <211> 30
   <212> DNA
   <213> artificial sequences
<220>
   <221> source
   <222> 1..30
   <223> /mol_type="DNA" /note="stem example" /organism="artificial sequences"
<400> 22
   tacggcagct gagtcaacag gttcttcccg 30
<210> 23
   <211> 30
   <212> DNA
   <213> artificial sequences
<220>
   <221> source
   <222> 1..30
   <223> /mol_type="DNA" /note="stem example" /organism="artificial sequences"
<400> 23
   gacggcagct gagtcaacag gttcttccca 30
<210> 24
   <211> 30
   <212> DNA
   <213> artificial sequences
<220>
   <221> source
   <222> 1..30
   <223> /mol_type="DNA" /note="stem example" /organism="artificial sequences"
<400> 24
   gacggcagct gagtcaacag gttcttcccc 30
<210> 25
   <211> 30
   <212> DNA
   <213> artificial sequences
<220>
   <221> source
   <222> 1..30
   <223> /mol_type="DNA" /note="stem example" /organism="artificial sequences"
<400> 25
   gacggcagct gagtcaacag gttcttccct 30
<210> 26
   <211> 30
   <212> DNA
   <213> artificial sequences
<220>
   <221> source
   <222> 1..30
   <223> /mol_type="DNA" /note="stem example" /organism="artificial sequences"
<400> 26
   gacggcagct gagtcaacag gttcttcccg 30
<210> 27
   <211> 28
   <212> DNA
   <213> artificial sequences
<220>
   <221> source
   <222> 1..28
   <223> /mol_type="DNA" /note="partial inflection example" /organism="artificial sequences"
<400> 27
   acggcagctg agtcaacagg ttcttccc 28
<210> 28
   <211> 28
   <212> DNA
   <213> artificial sequences
<220>
   <221> source
   <222> 1..28
   <223> /mol_type="DNA" /note="partial inflection example" /organism="artificial sequences"
<400> 28
   acggcagctg agtcaacagg ttcttccc 28
<210> 29
   <211> 29
   <212> DNA
   <213> artificial sequences
<220>
   <221> source
   <222> 1..29
   <223> /mol_type="DNA" /note="partial inflection example" /organism="artificial sequences"
<400> 29
   aacggcagct gagtcaacag gttcttccc 29
<210> 30
   <211> 29
   <212> DNA
   <213> artificial sequences
<220>
   <221> source
   <222> 1..29
   <223> /mol_type="DNA" /note="partial inflection example" /organism="artificial sequences"
<400> 30
   cacggcagct gagtcaacag gttcttccc 29
<210> 31
   <211> 29
   <212> DNA
   <213> artificial sequences
<220>
   <221> source
   <222> 1..29
   <223> /mol_type="DNA" /note="partial inflection example" /organism="artificial sequences"
<400> 31
   tacggcagct gagtcaacag gttcttccc 29
<210> 32
   <211> 29
   <212> DNA
   <213> artificial sequences
<220>
   <221> source
   <222> 1..29
   <223> /mol_type="DNA" /note="partial inflectin example" /organism="artificial sequences"
<400> 32
   gacggcagct gagtcaacag gttcttccc 29
<210> 33
   <211> 29
   <212> DNA
   <213> artificial sequences
<220>
   <221> source
   <222> 1..29
   <223> /mol_type="DNA" /note="partial inflection example" /organism="artificial sequences"
<400> 33
   acggcagctg agtcaacagg ttcttccca 29
<210> 34
   <211> 29
   <212> DNA
   <213> artificial sequences
<220>
   <221> source
   <222> 1..29
   <223> /mol_type="DNA" /note="partial inflection example" /organism="artificial sequences"
<400> 34
   acggcagctg agtcaacagg ttcttcccc 29
<210> 35
   <211> 29
   <212> DNA
   <213> artificial sequences
<220>
   <221> source
   <222> 1..29
   <223> /mol_type="DNA" /note="partial inflection example" /organism="artificial sequences"
<400> 35
   acggcagctg agtcaacagg ttcttccct 29
<210> 36
   <211> 29
   <212> DNA
   <213> artificial sequences
<220>
   <221> source
   <222> 1..29
   <223> /mol_type="DNA" /note="partial inflection example" /organism="artificial sequences"
<400> 36
   acggcagctg agtcaacagg ttcttcccg 29
<210> 37
   <211> 61
   <212> DNA
   <213> artificial sequences
<220>
   <221> source
   <222> 1..61
   <223> /mol_type="DNA" /note="example sequence" /organism="artificial sequences"
<400> 37
   cacggcagct gagtcaacag gttcttccca ggagcggacg gcggtggcca gcaggcacgt 60
   g 61
<210> 38
   <211> 30
   <212> DNA
   <213> artificial sequences
<220>
   <221> source
   <222> 1..30
   <223> /mol_type="DNA" /note="example sequence" /organism="artificial sequences"
<400> 38
   cacggcagct gagtcaacag gttcttccca 30
<210> 39
   <211> 30
   <212> DNA
   <213> artificial sequences
<220>
   <221> source
   <222> 1..30
   <223> /mol_type="DNA" /note="example sequence" /organism="artificial sequences"
<400> 39
   acggcagctg agtcaacagg ttcttcccag 30
<210> 40
   <211> 30
   <212> DNA
   <213> artificial sequences
<220>
   <221> source
   <222> 1..30
   <223> /mol_type="DNA" /note="example sequence" /organism="artificial sequences"
<400> 40
   cggcagctga gtcaacaggt tcttcccagg 30
<210> 41
   <211> 30
   <212> DNA
   <213> artificial sequences
<220>
   <221> source
   <222> 1..30
   <223> /mol_type="DNA" /note="example sequence" /organism="artificial sequences"
<400> 41
   ggcagctgag tcaacaggtt cttcccagga 30
<210> 42
   <211> 30
   <212> DNA
   <213> artificial sequences
<220>
   <221> source
   <222> 1..30
   <223> /mol_type="DNA" /note="example sequence" /organism="artificial sequences"
<400> 42
   gcagctgagt caacaggttc ttcccaggag 30
<210> 43
   <211> 30
   <212> DNA
   <213> artificial sequences
<220>
   <221> source
   <222> 1..30
   <223> /mol_type="DNA" /note="example sequence" /organism="artificial sequences"
<400> 43
   cagctgagtc aacaggttct tcccaggagc 30
<210> 44
   <211> 30
   <212> DNA
   <213> artificial sequences
<220>
   <221> source
   <222> 1..30
   <223> /mol_type="DNA" /note="example sequence" /organism="artificial sequences"
<400> 44
   agctgagtca acaggttctt cccaggagcg 30
<210> 45
   <211> 30
   <212> DNA
   <213> artificial sequences
<220>
   <221> source
   <222> 1..30
   <223> /mol_type="DNA" /note="example sequnece" /organism="artificial sequences"
<400> 45
   gctgagtcaa caggttcttc ccaggagcgg 30
<210> 46
   <211> 30
   <212> DNA
   <213> artificial sequences
<220>
   <221> source
   <222> 1..30
   <223> /mol_type="DNA" /note="example sequence" /organism="artificial sequences"
<400> 46
   ctgagtcaac aggttcttcc caggagcgga 30
<210> 47
   <211> 30
   <212> DNA
   <213> artificial sequences
<220>
   <221> source
   <222> 1..30
   <223> /mol_type="DNA" /note="example sequence" /organism="artificial sequences"
<400> 47
   tgagtcaaca ggttcttccc aggagcggac 30
<210> 48
   <211> 30
   <212> DNA
   <213> artificial sequences
<220>
   <221> source
   <222> 1..30
   <223> /mol_type="DNA" /note="example sequence" /organism="artificial sequences"
<400> 48
   gagtcaacag gttcttccca ggagcggacg 30
<210> 49
   <211> 30
   <212> DNA
   <213> artificial sequences
<220>
   <221> source
   <222> 1..30
   <223> /mol_type="DNA" /note="example sequence" /organism="artificial sequences"
<400> 49
   agtcaacagg ttcttcccag gagcggacgg 30
<210> 50
   <211> 30
   <212> DNA
   <213> artificial sequences
<220>
   <221> source
   <222> 1..30
   <223> /mol_type="DNA" /note="example sequence" /organism="artificial sequences"
<400> 50
   gtcaacaggt tcttcccagg agcggacggc 30
<210> 51
   <211> 30
   <212> DNA
   <213> artificial sequences
<220>
   <221> source
   <222> 1..30
   <223> /mol_type="DNA" /note="example sequence" /organism="artificial sequences"
<400> 51
   tcaacaggtt cttcccagga gcggacggcg 30
<210> 52
   <211> 30
   <212> DNA
   <213> artificial sequences
<220>
   <221> source
   <222> 1..30
   <223> /mol_type="DNA" /note="example sequence" /organism="artificial sequences"
<400> 52
   caacaggttc ttcccaggag cggacggcgg 30
<210> 53
   <211> 30
   <212> DNA
   <213> artificial sequences
<220>
   <221> source
   <222> 1..30
   <223> /mol_type="DNA" /note="example sequence" /organism="artificial sequences"
<400> 53
   aacaggttct tcccaggagc ggacggcggt 30
<210> 54
   <211> 30
   <212> DNA
   <213> artificial sequences
<220>
   <221> source
   <222> 1..30
   <223> /mol_type="DNA" /note="example sequence" /organism="artificial sequences"
<400> 54
   acaggttctt cccaggagcg gacggcggtg 30
<210> 55
   <211> 30
   <212> DNA
   <213> artificial sequences
<220>
   <221> source
   <222> 1..30
   <223> /mol_type="DNA" /note="example sequence" /organism="artificial sequences"
<400> 55
   caggttcttc ccaggagcgg acggcggtgg 30
<210> 56
   <211> 30
   <212> DNA
   <213> artificial sequences
<220>
   <221> source
   <222> 1..30
   <223> /mol_type="DNA" /note="example sequence" /organism="artificial sequences"
<400> 56
   aggttcttcc caggagcgga cggcggtggc 30
<210> 57
   <211> 30
   <212> DNA
   <213> artificial sequences
<220>
   <221> source
   <222> 1..30
   <223> /mol_type="DNA" /note="example sequence" /organism="artificial sequences"
<400> 57
   ggttcttccc aggagcggac ggcggtggcc 30
<210> 58
   <211> 30
   <212> DNA
   <213> artificial sequences
<220>
   <221> source
   <222> 1..30
   <223> /mol_type="DNA" /note="example sequence" /organism="artificial sequences"
<400> 58
   gttcttccca ggagcggacg gcggtggcca 30
<210> 59
   <211> 30
   <212> DNA
   <213> artificial sequences
<220>
   <221> source
   <222> 1..30
   <223> /mol_type="DNA" /note="example sequence" /organism="artificial sequences"
<400> 59
   ttcttcccag gagcggacgg cggtggccag 30
<210> 60
   <211> 30
   <212> DNA
   <213> artificial sequences
<220>
   <221> source
   <222> 1..30
   <223> /mol_type="DNA" /note="example sequence" /organism="artificial sequences"
<400> 60
   tcttcccagg agcggacggc ggtggccagc 30
<210> 61
   <211> 30
   <212> DNA
   <213> artificial sequences
<220>
   <221> source
   <222> 1..30
   <223> /mol_type="DNA" /note="example sequence" /organism="artificial sequences"
<400> 61
   cttcccagga gcggacggcg gtggccagca 30
<210> 62
   <211> 30
   <212> DNA
   <213> artificial sequences
<220>
   <221> source
   <222> 1..30
   <223> /mol_type="DNA" /note="example sequence" /organism="artificial sequences"
<400> 62
   ttcccaggag cggacggcgg tggccagcag 30
<210> 63
   <211> 30
   <212> DNA
   <213> artificial sequences
<220>
   <221> source
   <222> 1..30
   <223> /mol_type="DNA" /note="example sequence" /organism="artificial sequences"
<400> 63
   tcccaggagc ggacggcggt ggccagcagg 30
<210> 64
   <211> 30
   <212> DNA
   <213> artificial sequences
<220>
   <221> source
   <222> 1..30
   <223> /mol_type="DNA" /note="example sequence" /organism="artificial sequences"
<400> 64
   cccaggagcg gacggcggtg gccagcaggc 30
<210> 65
   <211> 30
   <212> DNA
   <213> artificial sequences
<220>
   <221> source
   <222> 1..30
   <223> /mol_type="DNA" /note="example sequence" /organism="artificial sequences"
<400> 65
   ccaggagcgg acggcggtgg ccagcaggca 30
<210> 66
   <211> 30
   <212> DNA
   <213> artificial sequences
<220>
   <221> source
   <222> 1..30
   <223> /mol_type="DNA" /note="example sequence" /organism="artificial sequences"
<400> 66
   caggagcgga cggcggtggc cagcaggcac 30
<210> 67
   <211> 30
   <212> DNA
   <213> artificial sequences
<220>
   <221> source
   <222> 1..30
   <223> /mol_type="DNA" /note="example sequence" /organism="artificial sequences"
<400> 67
   aggagcggac ggcggtggcc agcaggcacg 30
<210> 68
   <211> 30
   <212> DNA
   <213> artificial sequences
<220>
   <221> source
   <222> 1..30
   <223> /mol_type="DNA" /note="example sequence" /organism="artificial sequences"
<400> 68
   ggagcggacg gcggtggcca gcaggcacgt 30
<210> 69
   <211> 30
   <212> DNA
   <213> artificial sequences
<220>
   <221> source
   <222> 1..30
   <223> /mol_type="DNA" /note="example sequence" /organism="artificial sequences"
<400> 69
   gagcggacgg cggtggccag caggcacgtg 30
<210> 70
   <211> 30
   <212> DNA
   <213> artificial sequences
<220>
   <221> source
   <222> 1..30
   <223> /mol_type="DNA" /note="example sequence" /organism="artificial sequences"
<400> 70
   acggcagctg agtcaacagg ttcttcccag 30
<210> 71
   <211> 30
   <212> DNA
   <213> artificial sequences
<220>
   <221> source
   <222> 1..30
   <223> /mol_type="DNA" /note="example sequence" /organism="artificial sequences"
<400> 71
   cggcagctga gtcaacaggt tcttcccagg 30
<210> 72
   <211> 30
   <212> DNA
   <213> artificial sequences
<220>
   <221> source
   <222> 1..30
   <223> /mol_type="DNA" /note="example sequence" /organism="artificial sequences"
<400> 72
   ggcagctgag tcaacaggtt cttcccagga 30
<210> 73
   <211> 30
   <212> DNA
   <213> artificial sequences
<220>
   <221> source
   <222> 1..30
   <223> /mol_type="DNA" /note="example sequence" /organism="artificial sequences"
<400> 73
   gcagctgagt caacaggttc ttcccaggag 30
<210> 74
   <211> 30
   <212> DNA
   <213> artificial sequences
<220>
   <221> source
   <222> 1..30
   <223> /mol_type="DNA" /note="example sequence" /organism="artificial sequences"
<400> 74
   cagctgagtc aacaggttct tcccaggagc 30
<210> 75
   <211> 30
   <212> DNA
   <213> artificial sequences
<220>
   <221> source
   <222> 1..30
   <223> /mol_type="DNA" /note="example sequence" /organism="artificial sequences"
<400> 75
   agctgagtca acaggttctt cccaggagcg 30
<210> 76
   <211> 61
   <212> DNA
   <213> artificial sequences
<220>
   <221> source
   <222> 1..61
   <223> /mol_type="DNA" /note="example sequence" /organism="artificial sequences"
<400> 76
   cacggcagct gagtcaacag gttcttccca ggagcggacg gcggtggcca gcaggcacgt      60
   g                                                                      61
<210> 77
   <211> 29
   <212> DNA
   <213> artificial sequences
<220>
   <221> source
   <222> 1..29
   <223> /mol_type="DNA" /note="example sequence" /organism="artificial sequences"
<400> 77
   cacggcagct gagtcaacag gttcttccc 29
<210> 78
   <211> 29
   <212> DNA
   <213> artificial sequences
<220>
   <221> source
   <222> 1..29
   <223> /mol_type="DNA" /note="example sequence" /organism="artificial sequences"
<400> 78
   acggcagctg agtcaacagg ttcttccca 29
<210> 79
   <211> 29
   <212> DNA
   <213> artificial sequences
<220>
   <221> source
   <222> 1..29
   <223> /mol_type="DNA" /note="example sequence" /organism="artificial sequences"
<400> 79
   cggcagctga gtcaacaggt tcttcccag 29
<210> 80
   <211> 29
   <212> DNA
   <213> artificial sequences
<220>
   <221> source
   <222> 1..29
   <223> /mol_type="DNA" /note="example sequence" /organism="artificial sequences"
<400> 80
   ggcagctgag tcaacaggtt cttcccagg 29
<210> 81
   <211> 29
   <212> DNA
   <213> artificial sequences
<220>
   <221> source
   <222> 1..29
   <223> /mol_type="DNA" /note="example sequence" /organism="artificial sequences"
<400> 81
   gcagctgagt caacaggttc ttcccagga 29
<210> 82
   <211> 29
   <212> DNA
   <213> artificial sequences
<220>
   <221> source
   <222> 1..29
   <223> /mol_type="DNA" /note="example sequence" /organism="artificial sequences"
<400> 82
   cagctgagtc aacaggttct tcccaggag 29
<210> 83
   <211> 29
   <212> DNA
   <213> artificial sequences
<220>
   <221> source
   <222> 1..29
   <223> /mol_type="DNA" /note="example sequence" /organism="artificial sequences"
<400> 83
   agctgagtca acaggttctt cccaggagc 29
<210> 84
   <211> 29
   <212> DNA
   <213> artificial sequences
<220>
   <221> source
   <222> 1..29
   <223> /mol_type="DNA" /note="example sequence" /organism="artificial sequences"
<400> 84
   gctgagtcaa caggttcttc ccaggagcg 29
<210> 85
   <211> 29
   <212> DNA
   <213> artificial sequences
<220>
   <221> source
   <222> 1..29
   <223> /mol_type="DNA" /note="example sequence" /organism="artificial sequences"
<400> 85
   ctgagtcaac aggttcttcc caggagcgg 29
<210> 86
   <211> 29
   <212> DNA
   <213> artificial sequences
<220>
   <221> source
   <222> 1..29
   <223> /mol_type="DNA" /note="example sequence" /organism="artificial sequences"
<400> 86
   tgagtcaaca ggttcttccc aggagcgga 29
<210> 87
   <211> 29
   <212> DNA
   <213> artificial sequences
<220>
   <221> source
   <222> 1..29
   <223> /mol_type="DNA" /note="example sequence" /organism="artificial sequences"
<400> 87
   gagtcaacag gttcttccca ggagcggac 29
<210> 88
   <211> 29
   <212> DNA
   <213> artificial sequences
<220>
   <221> source
   <222> 1..29
   <223> /mol_type="DNA" /note="example sequence" /organism="artificial sequences"
<400> 88
   agtcaacagg ttcttcccag gagcggacg 29
<210> 89
   <211> 29
   <212> DNA
   <213> artificial sequences
<220>
   <221> source
   <222> 1..29
   <223> /mol_type="DNA" /note="example sequence" /organism="artificial sequences"
<400> 89
   gtcaacaggt tcttcccagg agcggacgg 29
<210> 90
   <211> 29
   <212> DNA
   <213> artificial sequences
<220>
   <221> source
   <222> 1..29
   <223> /mol_type="DNA" /note="example sequence" /organism="artificial sequences"
<400> 90
   tcaacaggtt cttcccagga gcggacggc 29
<210> 91
   <211> 29
   <212> DNA
   <213> artificial sequences
<220>
   <221> source
   <222> 1..29
   <223> /mol_type="DNA" /note="example sequence" /organism="artificial sequences"
<400> 91
   caacaggttc ttcccaggag cggacggcg 29
<210> 92
   <211> 29
   <212> DNA
   <213> artificial sequences
<220>
   <221> source
   <222> 1..29
   <223> /mol_type="DNA" /note="example sequence" /organism="artificial sequences"
<400> 92
   aacaggttct tcccaggagc ggacggcgg 29
<210> 93
   <211> 29
   <212> DNA
   <213> artificial sequences
<220>
   <221> source
   <222> 1..29
   <223> /mol_type="DNA" /note="example sequence" /organism="artificial sequences"
<400> 93
   acaggttctt cccaggagcg gacggcggt 29
<210> 94
   <211> 29
   <212> DNA
   <213> artificial sequences
<220>
   <221> source
   <222> 1..29
   <223> /mol_type="DNA" /note="example sequence" /organism="artificial sequences"
<400> 94
   caggttcttc ccaggagcgg acggcggtg 29
<210> 95
   <211> 29
   <212> DNA
   <213> artificial sequences
<220>
   <221> source
   <222> 1..29
   <223> /mol_type="DNA" /note="example sequence" /organism="artificial sequences"
<400> 95
   aggttcttcc caggagcgga cggcggtgg 29
<210> 96
   <211> 29
   <212> DNA
   <213> artificial sequences
<220>
   <221> source
   <222> 1..29
   <223> /mol_type="DNA" /note="example sequence" /organism="artificial sequences"
<400> 96
   ggttcttccc aggagcggac ggcggtggc 29
<210> 97
   <211> 29
   <212> DNA
   <213> artificial sequences
<220>
   <221> source
   <222> 1..29
   <223> /mol_type="DNA" /note="example sequence" /organism="artificial sequences"
<400> 97
   gttcttccca ggagcggacg gcggtggcc 29
<210> 98
   <211> 29
   <212> DNA
   <213> artificial sequences
<220>
   <221> source
   <222> 1..29
   <223> /mol_type="DNA" /note="example sequence" /organism="artificial sequences"
<400> 98
   ttcttcccag gagcggacgg cggtggcca 29
<210> 99
   <211> 29
   <212> DNA
   <213> artificial sequences
<220>
   <221> source
   <222> 1..29
   <223> /mol_type="DNA" /note="example sequence" /organism="artificial sequences"
<400> 99
   tcttcccagg agcggacggc ggtggccag 29
<210> 100
   <211> 29
   <212> DNA
   <213> artificial sequences
<220>
   <221> source
   <222> 1..29
   <223> /mol_type="DNA" /note="example sequence" /organism="artificial sequences"
<400> 100
   cttcccagga gcggacggcg gtggccagc 29
<210> 101
   <211> 29
   <212> DNA
   <213> artificial sequences
<220>
   <221> source
   <222> 1..29
   <223> /mol_type="DNA" /note="example sequence" /organism="artificial sequences"
<400> 101
   ttcccaggag cggacggcgg tggccagca 29
<210> 102
   <211> 29
   <212> DNA
   <213> artificial sequences
<220>
   <221> source
   <222> 1..29
   <223> /mol_type="DNA" /note="example sequence" /organism="artificial sequences"
<400> 102
   tcccaggagc ggacggcggt ggccagcag 29
<210> 103
   <211> 29
   <212> DNA
   <213> artificial sequences
<220>
   <221> source
   <222> 1..29
   <223> /mol_type="DNA" /note="example sequence" /organism="artificial sequences"
<400> 103
   cccaggagcg gacggcggtg gccagcagg 29
<210> 104
   <211> 29
   <212> DNA
   <213> artificial sequences
<220>
   <221> source
   <222> 1..29
   <223> /mol_type="DNA" /note="example sequence" /organism="artificial sequences"
<400> 104
   ccaggagcgg acggcggtgg ccagcaggc 29
<210> 105
   <211> 29
   <212> DNA
   <213> artificial sequences
<220>
   <221> source
   <222> 1..29
   <223> /mol_type="DNA" /note="example sequence" /organism="artificial sequences"
<400> 105
   caggagcgga cggcggtggc cagcaggca 29
<210> 106
   <211> 29
   <212> DNA
   <213> artificial sequences
<220>
   <221> source
   <222> 1..29
   <223> /mol_type="DNA" /note="example sequence" /organism="artificial sequences"
<400> 106
   aggagcggac ggcggtggcc agcaggcac 29
<210> 107
   <211> 29
   <212> DNA
   <213> artificial sequences
<220>
   <221> source
   <222> 1..29
   <223> /mol_type="DNA" /note="example sequence" /organism="artificial sequences"
<400> 107
   ggagcggacg gcggtggcca gcaggcacg 29
<210> 108
   <211> 29
   <212> DNA
   <213> artificial sequences
<220>
   <221> source
   <222> 1..29
   <223> /mol_type="DNA" /note="example sequence" /organism="artificial sequences"
<400> 108
   gagcggacgg cggtggccag caggcacgt 29
<210> 109
   <211> 29
   <212> DNA
   <213> artificial sequences
<220>
   <221> source
   <222> 1..29
   <223> /mol_type="DNA" /note="example sequence" /organism="artificial sequences"
<400> 109
   acggcagctg agtcaacagg ttcttccca 29
<210> 110
   <211> 30
   <212> DNA
   <213> artificial sequences
<220>
   <221> source
   <222> 1..30
   <223> /mol_type="DNA" /note="example sequence" /organism="artificial sequences"
<400> 110
   acggcagctg agtcaacagg ttcttcccaa 30
<210> 111
   <211> 30
   <212> DNA
   <213> artificial sequences
<220>
   <221> source
   <222> 1..30
   <223> /mol_type="DNA" /note="example sequence" /organism="artificial sequences"
<400> 111
   acggcagctg agtcaacagg ttcttcccac 30
<210> 112
   <211> 30
   <212> DNA
   <213> artificial sequences
<220>
   <221> source
   <222> 1..30
   <223> /mol_type="DNA" /note="example sequence" /organism="artificial sequences"
<400> 112
   acggcagctg agtcaacagg ttcttcccat 30
<210> 113
   <211> 30
   <212> DNA
   <213> artificial sequences
<220>
   <221> source
   <222> 1..30
   <223> /mol_type="DNA" /note="example sequence" /organism="artificial sequences"
<400> 113
   acggcagctg agtcaacagg ttcttcccag 30
<210> 114
   <211> 30
   <212> DNA
   <213> artificial sequences
<220>
   <221> source
   <222> 1..30
   <223> /mol_type="DNA" /note="example sequence" /organism="artificial sequences"
<400> 114
   agtcaacagg ttcttcccag gagcggacgc 30
<210> 115
   <211> 61
   <212> DNA
   <213> artificial sequences
<220>
   <221> source
   <222> 1..61
   <223> /mol_type="DNA" /note="example sequence" /organism="artificial sequences"
<400> 115
   cacggcagct gagtcaacag gttcttccca ggagcggacg gcggtggcca gcaggcacgt 60
   g 61
<210> 116
   <211> 61
   <212> DNA
   <213> artificial sequences
<220>
   <221> source
   <222> 1..61
   <223> /mol_type="DNA" /note="example sequence" /organism="artificial sequences"
<400> 116
   agcaggcacg tgacggcagc tgcagtcaac aggttcttcc caggagcgga cgccggtggc 60
   c 61
<210> 117
   <211> 61
   <212> DNA
   <213> artificial sequences
<220>
   <221> source
   <222> 1..61
   <223> /mol_type="DNA" /note="example read n. 1" /organism="artificial sequences"
<400> 117
   cacggcagct gagtcaacag gttcttccca ggagcggacg gcggtggcca gcaggcacgt 60
   g 61
<210> 118
   <211> 30
   <212> DNA
   <213> artificial sequences
<220>
   <221> source
   <222> 1..30
   <223> /mol_type="DNA" /note="example sequence" /organism="artificial sequences"
<400> 118
   acggcagctg agtcaacagg ttcttcccag 30
<210> 119
   <211> 30
   <212> DNA
   <213> artificial sequences
<220>
   <221> source
   <222> 1..30
   <223> /mol_type="DNA" /note="example sequence" /organism="artificial sequences"
<400> 119
   ggcagctgag tcaacaggtt cttcccagga 30
<210> 120
   <211> 30
   <212> DNA
   <213> artificial sequences
<220>
   <221> source
   <222> 1..30
   <223> /mol_type="DNA" /note="example sequence" /organism="artificial sequences"
<400> 120
   gcagctgagt caacaggttc ttcccaggag 30
<210> 121
   <211> 30
   <212> DNA
   <213> artificial sequences
<220>
   <221> source
   <222> 1..30
   <223> /mol_type="DNA" /note="example sequence" /organism="artificial sequences"
<400> 121
   cagctgagtc aacaggttct tcccaggagc 30
<210> 122
   <211> 30
   <212> DNA
   <213> artificial sequences
<220>
   <221> source
   <222> 1..30
   <223> /mol_type="DNA" /note="example sequence" /organism="artificial sequences"
<400> 122
   agctgagtca acaggttctt cccaggagcg 30
<210> 123
   <211> 30
   <212> DNA
   <213> artificial sequences
<220>
   <221> source
   <222> 1..30
   <223> /mol_type="DNA" /note="example sequence" /organism="artificial sequences"
<400> 123
   gctgagtcaa caggttcttc ccaggagcgg 30
<210> 124
   <211> 30
   <212> DNA
   <213> artificial sequences
<220>
   <221> source
   <222> 1..30
   <223> /mol_type="DNA" /note="example sequence" /organism="artificial sequences"
<400> 124
   tgagtcaaca ggttcttccc aggagcggac 30
<210> 125
   <211> 30
   <212> DNA
   <213> artificial sequences
<220>
   <221> source
   <222> 1..30
   <223> /mol_type="DNA" /note="example sequence" /organism="artificial sequences"
<400> 125
   gagtcaacag gttcttccca ggagcggacg 30
<210> 126
   <211> 30
   <212> DNA
   <213> artificial sequences
<220>
   <221> source
   <222> 1..30
   <223> /mol_type="DNA" /note="example sequence" /organism="artificial sequences"
<400> 126
   agtcaacagg ttcttcccag gagcggacgg 30
<210> 127
   <211> 61
   <212> DNA
   <213> artificial sequences
<220>
   <221> source
   <222> 1..61
   <223> /mol_type="DNA" /note="example read n. 2" /organism="artificial sequences"
<400> 127
   agcaggcacg tgacggcagc tgcagtcaac aggttcttcc caggagcgga cgccggtggc 60
   c 61
<210> 128
   <211> 30
   <212> DNA
   <213> artificial sequences
<220>
   <221> source
   <222> 1..30
   <223> /mol_type="DNA" /note="example sequence" /organism="artificial sequences"
<400> 128
   agtcaacagg ttcttcccag gagcggacgc 30
<210> 129
   <211> 61
   <212> DNA
   <213> artificial sequences
<220>
   <221> source
   <222> 1..61
   <223> /mol_type="DNA" /note="example leading read" /organism="artificial sequences"
<400> 129
   cacggcagct gagtcaacag gttcttccca ggagcggacg gcggtggcca gcaggcacgt 60
   g 61
<210> 130
   <211> 29
   <212> DNA
   <213> artificial sequences
<220>
   <221> source
   <222> 1..29
   <223> /mol_type="DNA" /note="example sequence" /organism="artificial sequences"
<400> 130
   acggcagctg agtcaacagg ttcttccca 29
<210> 131
   <211> 29
   <212> DNA
   <213> artificial sequences
<220>
   <221> source
   <222> 1..29
   <223> /mol_type="DNA" /note="example sequence" /organism="artificial sequences"
<400> 131
   ggcagctgag tcaacaggtt cttcccagg 29
<210> 132
   <211> 29
   <212> DNA
   <213> artificial sequences
<220>
   <221> source
   <222> 1..29
   <223> /mol_type="DNA" /note="example sequence" /organism="artificial sequences"
<400> 132
   gcagctgagt caacaggttc ttcccagga 29
<210> 133
   <211> 29
   <212> DNA
   <213> artificial sequences
<220>
   <221> source
   <222> 1..29
   <223> /mol_type="DNA" /note="example sequence" /organism="artificial sequences"
<400> 133
   cagctgagtc aacaggttct tcccaggag 29
<210> 134
   <211> 29
   <212> DNA
   <213> artificial sequences
<220>
   <221> source
   <222> 1..29
   <223> /mol_type="DNA" /note="example sequence" /organism="artificial sequences"
<400> 134
   agctgagtca acaggttctt cccaggagc 29
<210> 135
   <211> 29
   <212> DNA
   <213> artificial sequences
<220>
   <221> source
   <222> 1..29
   <223> /mol_type="DNA" /note="example sequence" /organism="artificial sequences"
<400> 135
   gctgagtcaa caggttcttc ccaggagcg 29
<210> 136
   <211> 29
   <212> DNA
   <213> artificial sequences
<220>
   <221> source
   <222> 1..29
   <223> /mol_type="DNA" /note="example sequence" /organism="artificial sequences"
<400> 136
   tgagtcaaca ggttcttccc aggagcgga 29
<210> 137
   <211> 29
   <212> DNA
   <213> artificial sequences
<220>
   <221> source
   <222> 1..29
   <223> /mol_type="DNA" /note="example sequence" /organism="artificial sequences"
<400> 137
   gagtcaacag gttcttccca ggagcggac 29
<210> 138
   <211> 29
   <212> DNA
   <213> artificial sequences
<220>
   <221> source
   <222> 1..29
   <223> /mol_type="DNA" /note="example sequence" /organism="artificial sequences"
<400> 138
   agtcaacagg ttcttcccag gagcggacg 29
<210> 139
   <211> 61
   <212> DNA
   <213> artificial sequences
<220>
   <221> source
   <222> 1..61
   <223> /mol_type="DNA" /note="example sequence" /organism="artificial sequences"
<400> 139
   cacggcagct gagtcaacag gttcttccca ggagcggacg gcggtggcca gcaggcacgt 60
   g 61
<210> 140
   <211> 61
   <212> DNA
   <213> artificial sequences
<220>
   <221> source
   <222> 1..61
   <223> /mol_type="DNA" /note="example sequence" /organism="artificial sequences"
<400> 140
   agcaggcacg tgacggcagc tgcagtcaac aggttcttcc caggagcgga cgccggtggc 60
   c 61
<210> 141
   <211> 29
   <212> DNA
   <213> artificial sequences
<220>
   <221> source
   <222> 1..29
   <223> /mol_type="DNA" /note="example sequence" /organism="artificial sequences"
<400> 141
   agtcaacagg ttcttcccag gagcggacg 29
<210> 142
   <211> 30
   <212> DNA
   <213> artificial sequences
<220>
   <221> source
   <222> 1..30
   <223> /mol_type="DNA" /note="example sequence Table 1" /organism="artificial sequences"
<400> 142
   actgactgac tgactgactg actgactgaa 30
<210> 143
   <211> 30
   <212> DNA
   <213> artificial sequences
<220>
   <221> source
   <222> 1..30
   <223> /mol_type="DNA" /note="example sequence Table 1" /organism="artificial sequences"
<400> 143
   actgactgac tgactgactg actgactgac 30
<210> 144
   <211> 30
   <212> DNA
   <213> artificial sequences
<220>
   <221> source
   <222> 1..30
   <223> /mol_type="DNA" /note="example sequence Table 1" /organism="artificial sequences"
<400> 144
   actgactgac tgactgactg actgactgag 30
<210> 145
   <211> 30
   <212> DNA
   <213> artificial sequences
<220>
   <221> source
   <222> 1..30
   <223> /mol_type="DNA" /note="example sequence Table 1" /organism="artificial sequences"
<400> 145
   actgactgac tgactgactg actgactgat 30
<210> 146
   <211> 29
   <212> DNA
   <213> artificial sequences
<220>
   <221> source
   <222> 1..29
   <223> /mol_type="DNA" /note="example sequence Table 2" /organism="artificial sequences"
<400> 146
   actgactgac tgactgactg actgactga 29
<210> 147
   <211> 29
   <212> DNA
   <213> artificial sequences
<220>
   <221> source
   <222> 1..29
   <223> /mol_type="DNA" /note="example sequence Table 2" /organism="artificial sequences"
<400> 147
   actgactgac tgactgactg actgactgc 29

## Description

This application claims the benefit of European Patent Application EP16178577.9 filed July 8th, 2016.

The present invention relates to a computer implemented method for the identification and characterization of sequence variants in nucleic acids. In particular, this method is able to quickly and accurately identify most types of sequence genome variations with a potential association to a disease, that is, from single nucleotide substitutions to large structural variants. This method may have multiple and direct applications in genomics-based diagnosis, prognosis and therapy.

The invention further relates to a computer program and to systems suitable for performing such a method. The computer program may be designed to be lock-less and scalable, thereby allowing for high performance implementations on parallel execution environments such as specialized hardware accelerators.

### BACKGROUND ART

The genetic basis of disease is increasingly becoming more accessible thanks to the emergence of the Next Generation Sequencing (NGS) platforms, which have extremely reduced the costs and increased the throughput of genomic sequencing. For the first time in history, personalized medicine is close to becoming a reality through the analysis of each patient's genome.

A wide range of genome variation of cells and individuals has been identified to be the direct cause, or a predisposition to genetic diseases: from single nucleotide variants (SNVs if they are somatic, and SNPs if they are polymorphic in the population), to structural variants (SVs), which can correspond to deletions, insertions, inversions, translocations and copy number variations (CNVs), ranging from a few nucleotides to large genomic regions, including complete chromosome arms. These variations can exist between patients and also emerge among cells of the same patient. The unveiling of changes in the genome is driving discoveries such as the Philadelphia translocation between chromosomes 9 and 22, whose presence implies the development of chronic myelogenous leukemia (CML) and its identification allows the development and selection of last-generation therapies.

The ideal exploitation of genomic sequencing should involve the accurate identification of all variants, in order to derive a correct diagnosis and to select the best therapy. For clinical purposes, it is important that this computational process be carried out within an effective timeframe. But a simple sequencing experiment typically yields thousands of millions of reads per genome, which have to be stored and analysed. The task is severely hindered by a variety of factors such as PCR-amplification and sequencing errors, limitations intrinsically linked to the size of the reads, biases in the sequencing techniques employed, the inherent repetitive and dynamic nature of the genomic sequences, and others. As a consequence, the analysis of genomes with diagnostic and therapeutic purposes is still a great challenge, both in the design of efficient algorithms and at the level of computing performance.

Modern medicine will rely on the identification of genetic markers for precision diagnosis and for the application of more specific therapies. Cancer is one of the most active diagnostic and therapeutic areas where genetic analysis is being applied. Having access to all somatic variation accumulated in a tumor cell is now allowing the study of the genetic causes of the tumor and the development of new clinical protocols that are already starting to be applied in some clinical centres, and that will be soon a reality for all modern healthcare systems around the world. This is why, the identification of tumor variants is key in research and soon, also in medical care. The variants responsible for the origin and progression of tumors are currently searched using a common scheme that involves the sequencing of both tumor and normal genome samples of the same patient, and the subsequent scan and identification of the differences between them. Most of the available methods rely on an initial step, where all the normal and tumor sequence reads are aligned to a reference genome to then identify the changes present in the tumor compared with the normal and reference genomes. Despite these methods have provided a great number of disease-associated variation so far, they still entail intrinsic limitations associated to the need of a prealignment to a reference genome, affecting their performance and accuracy. More precisely, the reference-based identification of somatic variation in cancer genomes has currently the following sources of errors and limitations: (i) the initial alignment step, on which all the methods rely, is time consuming and particularly error prone with the tumor reads that carry the sequence variation, which are the most relevant for the analysis. It has been proven that many of these reads that carry changes and differences in their sequence are difficult or even impossible to align to the reference unmutated genome. The absence and the misplacement of tumor reads in the final alignment drastically affect all existing downstream methods for variant searching and calling. Although a number of alternative methods exist, this alignment step is generally performed with the same program (Li, H., et.al. "Fast and accurate short read alignment with Burrows-Wheeler transform" Bioinformatics 2009, vol. 25, pp. 1754-1760), which implies that nearly all analyses done nowadays share the same type of errors derived from this mapping of reads. (ii) The usage of a reference genome also involves the interference with millions of inherited variants (germline, i.e. not somatic) that affect both, the accuracy at the level of read mapping and the actual identification of the target somatic fraction (normally comprising only between 2 and 10 thousand variants). A considerable number of these germline variants are then frequently mispredicted as somatic changes, increasing the rate of false positives and decreasing, consequently, the final reliability and applicability of the results.

On top of the limitations and errors inherent to the generation and dependency of this initial alignment, the subsequent analysis, where somatic changes are finally identified, also implies a number of restrictions and complications. For example, despite the great deal of possibilities in terms of available methods, not a single one of them is able to identify a wide range of somatic variation, but instead, each is limited to the detection of a particular size and type of mutation (Medvedev P. "Computational methods for discovering structural variation with next-generation sequencing" Nat. Methods Suppl. 2009, vol. 6, S13-S20)There are programs that use this alignment to detect only SNVs and others that only identify SVs, among which, each one is able to detect a particular variant size. For instance, some methods identify insertions or deletions that comprise a few nucleotides (from 2 to a few dozens), others detect medium size SVs (from a few dozens of nucleotides to a few hundreds), and a small fraction of them, are designed for the identification of larger SVs (Ding, L., et.al. "Expanding the computational toolbox for mining cancer genomes" Nat. Rev. Genet. 2014, vol. 15, pp. 556-570). As the detection complexity increases with the detection range, methods designed for the identification of large SVs are also more imprecise in defining the exact location in the genome and the type of change, which are often necessary for being able to derive the functional consequences of the mutation. These programs often only report regions where SVs might be located.

In order to overcome these limitations, to date, alternative approximations have been developed. The term reference-free becoming more popular and has recently been used to describe a wide range of fundamentally different underlying strategies. For example, these methodologies are using fundamentally different unrelated strategies covering, from the use of reference mapping plus assembly-based (Chen K. "TIGRA: A targeted Iterative Graph Routing Assembler for Breakpoint Assembly" Genome Res. 2016, vol. 24, pp.310-317), *de novo* assembly (Zhuang, J. "Local sequence assembly reveals a high-resolution profile of somatic structural variations in 97 cancer genomes" Nucleic Acid Research 2015, vol. 43, pp.8146-8156), to suffix tree approximations (Moncunill V., et al., "Comprehensive characterization of complex structural variations by directly comparing genome sequence reads" Nature Biotech. 2014, vol. 32, pp. 1106-1112). The first two examples are based on the end-joining of reads in the tumor and normal genomes in order to identify discordant patterns. Although these assembly can also suffer the mapping-derived limitations, they have other major limitations associated to the underlying mechanism of the assembly, mostly when using NGS reads, as the overlapping regions needed to extend over the read size are often too small to be position-specific in the genome. Among other reference-free approximations reported to date, it can be highlighted a suffix tree-based method (SMUFIN) that compares in a tree-like structure all tumor and normal reads, to then extract discordant branches as candidate positions for variation. Although this particular way of analysing reads may directly overcome many of the limitations mentioned, it still lacks possibilities for detecting non-human sequences and is limited by the size of the tree, which grows in memory demands as sequencing coverage grows. Additionally, and in contrast to the approach followed in the method of the present invention, suffix trees are data structures that inherently require locking access patterns to allow for concurrent updates to take place, therefore limiting the ability to efficiently implement these approaches in high performance parallel computing systems. These two fundamentally different approaches of analysing sequence reads have also limitations of scalability, as the design of the code has not considered alternative ways of adapting to specific and more efficient hardware architectures. In fact, all the limitations mentioned hinder the incorporation of this type of genomic analysis into identification of somatic mutations applied to the clinical practice, which calls for much faster and more accurate computational methods. In addition, current methods for somatic variant calling still miss an important fraction of large SVs, which are relevant for the diagnosis and treatment of diseases.

Similar approximations have also been extended to deal with other type of problems in molecular biology. For example, some reference free methods have been developed to quantify the abundance of RNA isoforms from RNA-seq data (Patro R., et. al. "Sailfish enables alignment-free isoform quantification from RNA-seq reads using lightweight algorithms" Nature Biotech. 2014, vol. 32, pp. 462-464), or to identify evolutionary-driven substitutions in homozygosis, using *de novo* assembly of plant genomes (Nordstrom K.J.V, et.al. "Mutation identification by direct comparison of whole-genome sequencing data from mutant and wild-type individuals using k-mers" Nature Biotech. 2013, vol. 31, pp. 325-331).

Clearly quick and robust comparative methods, able to detect all kinds of SVs differentiating two states (normal vs. pathological, undifferentiated vs. differentiated, etc.) with high sensitivity, specificity, speed and scalability are still needed, as well as systems and computer programs suitable for performing such methods.

### SUMMARY OF THE INVENTION

In contrast to what is found in the *prior art,* inventors have come up with a computer-implemented method for identifying nucleic acid variants between two genomic states that does not depend on the alignment of reads of either state to a reference genome, or on the construction of sequence-based suffix trees. Using a different underlying mechanism, this method is, on its own, able to accurately identify all types of variants (heterozygous and homozygous), from single nucleotide variations to large structural variants at base pair resolution with unprecedented performance at the level of variant detection and execution possibilities. Importantly, the method is not restricted to the identification of a certain type of variant (SNVs, insertions, inversions, etc.) nor does it only perform for variants of a certain size, as is the case for many of the methods found in the art. Because of its underlying principle, based on the use of k-mers and hashtables independently of a reference genome, all the limitations that apply to all other existing methods (outlined above) are overcome. This translates to a method that is not only more robust, but is also more thorough and much faster than the methods described to date.

This invention entails a reference-free detection method that allows discovering homozygous and heterozygous variation in genomes using a polymorphic k-mer strategy consisting in the sequential sub-selection of read regions that will be compared in different ways to finally isolate variant-containing regions. One of the key elements of this computer-implemented method is the way k-mers are handled, as they are taken as "dynamic entities" by taking their stems and using the latter to explore for their inflections and partial inflections (see below). Compared to the other reference-free methods described above, the invention relies on a fundamentally different way of dealing with the reads since, instead of constructing an assembly, or a complete suffix-tree with all normal and tumor reads, it uses a particular k-mer strategy (see below) to fish reads with potential variation and discards, in one pass, the vast majority of reads with no information. This allows inventors to quickly filter and retain a subset of reads representing all the variants that are now computationally easy to treat and analyse.

Of note, the use of k-mers for direct comparison of genomes has only been explored in simple scenarios with a small scope, data, and requirements (see for instance Nordstrom K., *ibid*). In general, the use of k-mers has some limitations due to their strict nature, and the way k-mers are distributed in genomes, requiring large amounts of computing resources if the identification of unique features is sought. Inventors address these limitations by using a more flexible approach: polymorphic k-mers, which in addition to k-mers, also identify variations (inflections) of k-mers with similar patterns (stems, see below). Unlike regular, fixed-length k-mers, polymorphic k-mers enable the identification of unique features, and at the same time provide the means to gather and group related sequences even if they are not strictly the same. This element is key, as will be seen in the examples found below.

Thus, a first aspect of the present invention is a computer-implemented method for identifying nucleic acid variants between two genomic states comprising the steps of: A) Inputting 2 sets of nucleic acid reads, which are sequences retrieved from a nucleotide sequencing method, wherein the first set of reads corresponds to cells representing a first test state, and the second set of reads corresponds to cells representing a second control state; B) Filtering the reads, wherein the filtering comprises: B1) Keeping only the reads with at least a percentage X1 of their bases with a Phred quality score higher than 20, being X1 equal to or above 90%; B2) Splitting the reads with an undefined nucleotide, giving one sequence before, and one sequence after the undefined nucleotide, the latter being discarded; and B3) Discarding the sequence reads with less than X2 bases, wherein X2 is from 25 to 50; C) Generating a hashtable structure comprising: C1) Generating a number of N-X2+1 new reads for each read of sequence length N, wherein the new N-X2+1 reads correspond to all k-mers with length X2 nucleotides; and C2) Building a hashtable structure, which comprises all the k-mers generated in step C1) and further comprises the number of times each k-mer is observed in the two sets of reads corresponding to first and second states. D) Detecting candidate variants in the sequence between first state and second state, wherein a k-mer of the hashtable structure is taken as a candidate breakpoint, which represents a variant between the first and second states, if it fulfills all the following requirements: D1) At least one inflection based on a k-mer's stem must have at least X3 reads with the same variation between first and second states, being X3 at least 2; D2) The percentage of first state reads in second state reads is not over a threshold X4, to account for possible contamination of control state reads with test state reads, wherein X4 is at least 5%. E) Clustering and filtering test and control reads derived from all candidate breakpoints accepted in step D to build blocks, by carrying out the steps: E1) Retrieving reads which contain the stem of at least one k-mer that represents the candidate breakpoint selected in step D); E2) The reads of step E1) with at least X5 k-mer variants within a window of X6 nucleotides are taken as leading reads, wherein X5 is at least 7 and X6 is at least 10; E3) Reads whose k-mers share at least one stem with a leading read are merged to give a block; and E4) If the nucleic acid whose variant is being identified is a double stranded DNA, then both forward and reverse variants are taken into account when building the block. F) Aligning blocks taking their leading reads as a reference: F1) For each read in the block, take the leading read's stem and find the longest inflection or partial inflection between the read and the leading read. F2) Successively position each read so that its matching inflection or partial inflection is aligned against the leading read.

The performance and speed of this method make it more suitable for clinical applications (such as genomic analysis of cancer cells) than the alternative solutions, which result complex and time-consuming. As it is shown in the data below, the method has a superior performance even to last-generation methods such as the one published in Moncunill (*ibid*), which is also reference-free.

A second aspect of the present invention is a computer program product comprising program instructions for causing a computer system to perform the computer-implemented method for identifying nucleic acid variants between two genomic states of the first aspect of the invention.

The computer program product may be embodied on a storage medium (for example, a CD-ROM, a DVD, a USB drive, on a computer memory or on a read-only memory) or carried on a carrier signal (for example, on an electrical or optical carrier signal).

The computer program may be in the form of source code, object code, a code intermediate source and object code such as in partially compiled form, or in any other form suitable for use in the implementation of the processes according to the invention. The carrier may be any entity or device capable of carrying the computer program.

For example, the carrier may comprise a storage medium, such as a ROM, for example a CD ROM or a semiconductor ROM, or a magnetic recording medium, for example a floppy disc or hard disk. Further, the carrier may be a transmissible carrier such as an electrical or optical signal, which may be conveyed via electrical or optical cable or by radio or other means.

When the computer program is embodied in a signal that may be conveyed directly by a cable or other device or means, the carrier may be constituted by such cable or other device or means.

Alternatively, the carrier may be an integrated circuit in which the computer program is embedded, the integrated circuit being adapted for performing, or for use in the performance of, the relevant methods.

A third aspect of the invention is a system for identifying nucleic acid variants between two genomic states, the system comprising:
A) Computer/Electronic means for inputting 2 sets of nucleic acid reads, which are sequences retrieved from a nucleotide sequencing method, wherein the first set of reads corresponds to cells representing a first test state, and the second set of reads corresponds to cells representing a second control state;
B) Computer/Electronic means for filtering the reads, wherein the filtering comprises: B1) Keeping only the reads with at least a percentage X1 of their bases with a Phred quality score higher than 20, being X1 equal to or above 90%; B2) Splitting the reads with an undefined nucleotide, giving one sequence before, and one sequence after the undefined nucleotide, the latter being discarded; and B3) Discarding the sequence reads with less than X2 bases, wherein X2 is from 25 to 50; C) Computer/Electronic means for generating a hashtable structure comprising: C1) Generating a number of N-X2+1 new reads for each read of sequence length N, wherein the new N-X2+1 reads correspond to all k-mers with length X2 nucleotides; and
C2) Building a hashtable structure, which comprises all the k-mers generated in step C1) and further comprises the number of times each k-mer is observed in the two sets of reads corresponding to first and second states.
D) Computer/Electronic means for detecting variants in the sequence between first state and second state, wherein a k-mer of the hashtable structure is taken as a candidate breakpoint, which represents a variant between the first and second states, if it fulfills all the following requirements: D1) At least one inflection based on a k-mer's stem must have at least X3 reads with the same variation between first and second states, being X3 at least 2; D2) The percentage of first state reads in second state reads is not over a threshold X4, to account for possible contamination of control state reads with test state reads, wherein X4 is at least 5%. E) Computer/Electronic means for clustering and filtering test and control reads derived from all candidate breakpoints accepted in step D to build blocks, by carrying out the steps: E1) Retrieving reads which contain the stem of at least one k-mer that represents the candidate breakpoint selected in step D); E2) The reads of step E1) with at least X5 k-mer variants within a window of X6 nucleotides are taken as leading reads, wherein X5 is at least 7 and X6 is at least 10; E3) Reads whose k-mers share at least one stem with a leading read are merged to give a block; and E4) If the nucleic acid whose variant is being identified is a double stranded DNA, then both forward and reverse variants are taken into account when building the block. F) Computer/Electronic means for aligning blocks taking their leading reads as a reference: F1) For each read in the block, take the leading read's stem and find the longest inflection or partial inflection between the read and the leading read. F2) successively position each read so that its matching inflection or partial inflection is aligned against the leading read.

The electronic/computer means may be used interchangeably, that is, a part of the described means may be electronic means and the other part may be computer means, or all described means may be electronic means or all described means may be computer means. Examples of an apparatus comprising only electronic means may be a CPLD (Complex Programmable Logic Device), a FPGA (Field Programmable Gate Array) or an ASIC (Application-Specific Integrated Circuit).

A fourth aspect of the invention is a computer system comprising a processor and a memory, wherein the memory stores computer executable instructions that, when executed by the processor, cause the system to perform the method for identificatying nucleic acid variants between two genomic states. In some examples, the computer system may further comprise a hardware accelerator.

### BRIEF DESCRIPTION OF THE DRAWINGS

FIG1. Data flow for the proposed configuration of the system. 1.1 Quality Check; 1.2 Base Convertion; 1.3 Reduce; 1.4 Hash; 1.5 Orchestable Data Movement; 1.6 Store Data into Associative Structure; 1.7 Load Sequences and Quality Markers; 1.8 Network; 1.9 Disk(s).
FIG2. Filtering capabilities of the method at different stages, -o- is Total Reads; -x-is Supporting Reads; -I- is Identifiable Mutations.
FIG3. Visualization of a breaking-block with an insertion of virus

### DETAILED DESCRIPTION OF THE INVENTION

All terms as used herein, unless otherwise stated, shall be understood in their ordinary meaning as known in the art. Other more specific definitions for certain terms as used in the present application are as set forth below and are intended to apply uniformly throughout the description and claims unless an otherwise expressly set out definition provides a broader definition.

It must be noted that for clarity reasons, a variety of nucleotide sequences are given in the definitions and the examples found below. These nucleotide sequences are made up for these examples and they do not refer to any real nucleotide sequence of any organism. They are only listed so that the reader of the present application understands the terms used such as k-mer, stem, inflection, partial inflection, etc. They are absolutely unrelated to the invention being disclosed herewith, which has nothing to do with any particular nucleotide sequence.

The terms "computational method" and "computer implemented method" are taken here to mean the same and are used interchangeably. Therefore, "computational method" and "computer implemented method" are taken as synonyms.

The terms "Next Generation Sequencing" (NGS), "deep sequencing", "ultra-deep sequencing", "high throughput sequencing" are all used interchangeably and refer to the technology platforms currently being used as standard to enable the sequencing of genomes ("sequencing methods") with high speed and contained cost, such as the Roche/454, Illumina/Solexa, Life/APG and Pacific Biosciences platforms.

The term "base" and the term "nucleotide" are herein used interchangeably, and refer to the monomers (subunits) which are repeated in a nucleic acid such as DNA or RNA, giving its sequence or primary structure.

The term "reference genome" as used herein refers to the complete nucleic acid sequence representing the whole genome of a species normally accepted by the wide community. Since the reference genome is usually assembled from the sequencing of DNA from a number of donors, it does not accurately represent the set of genes of any one single individual. Instead, a reference genome provides a mosaic of different DNA sequences from each donor. But, at general levels, the reference genome provides a good approximation of the DNA of any single individual. However, in genomic regions with high allelic diversity, the reference genome may differ significantly from any one single individual. For example, GRCh37, the Genome Reference Consortium human genome (build 37) is derived from thirteen anonymous volunteers from New York. Reference genomes are typically used as a guide on which new genomes are built and aligned, enabling their assembly and comparison.

The term "forward strand" as used herein refers to a nucleic acid sequence read from 5' terminal to 3' terminal ends. The term "reverse strand" refers to the nucleic acid sequence which is complementary to the forward strand.

The term "nucleic acid variant" or simply "variant" as used herein refers to a difference in sequence between two genomic states. A variant can be a single nucleotide variant (SNV) if the difference between the two genomes (or two states of the same genome) is only due to the change of a single nucleotide. All other variants, among them insertions, deletions, inversions, duplications, translocations and others are termed structural variants (SV). The latter can have many sizes, from two bases up to entire pieces of a chromosome.

The term "genomic state" as used herein can refer to two different genomes derived from two different individuals, or two genomes derived from two different cells of the same individual. In the second case, the two different cells can be a normal vs. a pathological cell, an undifferentiated vs. a differentiated cell, a cell which has been exposed to a certain external factor vs. an unexposed cell, etc.

The term "mapping" as used herein refers to aligning blocks of the first and second state to a reference genome.

The term "read" as used herein refers to a fragment of nucleic acid that is sequenced in its entirety. The nucleic acid might be DNA, RNA, or even chemically altered nucleic acids. The initial step in a high throughput sequencing run is the random fragmentation of a genome into millions of partly overlapping fragments called reads, which are usually amplified by Polymerase Chain Reaction and sequenced using a variety of techniques that are platform-dependent. The lengths of the reads can also vary depending on the platform, and are usually on the order of a few dozens to a few hundreds of nucleotides. The partly overlapping reads must be assembled if a complete picture of the genome is to be built.

The term "depth of coverage" as used herein refers to the number of times a nucleotide is read during the sequencing process. Deep sequencing means that the total number of reads is many times larger than the length of the sequence under study. Standard depth of coverage currently range from 30x to 100x for whole genomes, meaning that each position in the genome is represented from 30 to 100 times. Coverage similarly designates the average number of reads representing a given nucleotide in the reconstructed sequence.

Depth of coverage can be calculated from the length of the original genome (G), the number of reads (*N*), and the average read length (L) as N*L/G.

The term "undefined nucleotide" as used herein refers to a certain position inside a sequenced read that could not be determined during the sequencing process, that is, a position for which the sequencing experiment has not unambiguously resolved whether it is occupied by an adenine (A), guanine (G), cytosine (C) or thymine (T), and therefore its nature is unknown. Undefined nucleotides in reads are filtered out (removed) in the method of the invention, generating two or more fragments of defined sequence if the undefined nucleotides are removed form inner positions of the read.

The term "Phred quality score" as used herein refers to the quality score given to each nucleotide base call in a sequenced read. The Phred score is a property given to each sequenced nucleotide and it is logarithmically related to the base-calling error probability. A Phred score of 10 assigned to a certain nucleotide in a sequenced read means that there is a 90% probability that the base call is correct, a Phred score of 20 means that there is a 99% probability that the base call is correct, and a Phred score of 30 means that there is a 99.9% probability that the base call is correct.

The term "assembling" as used herein refers to grouping all the first state reads, and separately second state reads that share the same variant.

The term "hashtable" as used herein refers to a data structure used in computing that allows (by applying a hash function) assigning and mapping hashes (values) to strings of data, that is, it associates a series of hashes (values) to a series of strings in pairs, such that the association of hash-string is established. The addition, removal and modification of pairs is easily achieved by computational means, as well as the lookup and accession of strings thanks to their respective hashes (values).

The term "hash" as used herein refers to the value given by the hash function and linked to a certain string. This value allows computationally storing, retrieving, deleting and sorting strings in a very efficient manner.

The term "hash function" as used herein refers to the function that is used for linking hashes (values given by the hash function) to strings of data given as input.

The term "k-mer" as used herein refers to all possible substrings of length k that are contained in a string. In genomics, all k-mers of a nucleic acid read are all the possible sub-sequences within the original read which have a length k. The amount of k-mers in a read of length M is M-k+1.

The term "polymorphic k-mer" as used herein refers to a k-mer that also identifies inflections and partial inflections of the k-mer's stem. By polymorphic k-mer it is here understood the way the method of the invention handles k-mers, that is, the way they are used to derive stems and the latter to search for, manipulate and fish inflections and partial inflections.

The term "stem" as used herein refers to a fragment of a k-mer of length k with S defined bases, where S < k, and k-S omitted (undefined) bases. The stem fragment can either be a k-mer without a prefix, a k-mer without a suffix, a k-mer without an infix, or any combination thereof. Stem fragments without infix and/or prefix are consecutive, while stems without infixes can be nonconsecutive. In a stem, the character "-" denotes a base that is omitted from the k-mer. Examples of the 30-mer SEQ ID NO: 1
CACGGCAGCTGAGTCAACAGGTTCTTCCCA:
SEQ ID NO:2
   CACGGCAGCTGAGTCAACAGGTTCTTCCC- (omission of suffix of length 1)
SEQ ID NO:3
   -ACGGCAGCTGAGTCAACAGGTTCTTCCC- (omission of prefix of length 1, and suffix of length 1)
SEQ ID NO:4
   CACG--AGCTGAGTCAACAGGTTCTTCCCA (omission of infix of length 2 starting at position 5)

The term "prefix" as used herein, refers to the first part of a sequencing read, that is, from position 1 to a given position depending on the context. This term is used here, as it is used in a grammatical context referring to words.

The term "suffix" as used herein, refers to the last part of a sequencing read, that is, from the last position to a given position depending on the context. This term is used here, as it is used in a grammatical context referring to words.

The term "infix" as used herein, refers to a part of a read positioned in the middle of the sequence.

The term "inflection" as used herein refers to a fragment of length k that can be derived from extending a stem of length k-1, k-2, k-3, etc. of a k-mer of length k. E.g. A stem of length k-1 can be used to derive 4 inflections of length k since there is a single unknown position, and 4 different bases (4¹=4). A stem of length k-2 can be used to derive 16 inflections of length k (4²=16). Following the example given above, for the stem (SEQ ID NO:5): "CACGGCAGCTGAGTCAACAGGTTCTTCCC-" (omission of suffix of length 1) the inflections would be (SEQ ID NO:6 to SEQ ID NO:9): and further,
inflections based on stem (SEQ ID NO:10)
"-ACGGCAGCTGAGTCAACAGGTTCTTCCC-" would be (SEQ ID NO:11-SEQ ID NO:26):

The term "partial inflection" as used herein refers to a fragment with P defined bases that can be derived from extending a stem of S defined bases of a k-mer of length k, and where S<=P<k. In a partial inflection, the «.» character denotes a non-extended position of its stem. Partial inflections must have at least one non-extended position. Only omitted bases («-») can be marked as non-extended.
Following the example given above:
Partial inflections based on stem (SEQ ID NO:27)
"-ACGGCAGCTGAGTCAACAGGTTCTTCCC-" would be (SEQ ID NO:28 - SEQ ID NO:36):

The term "breakpoint" as used herein refers to the the nucleotide position where the sequence changes, that is sequence immediately flanking a sequence variant. For SVs, a breakpoint is the point where the DNA broke in the second state and appears as a change in the first state compared to the second control state. In other words, where the continuity of the sequence of the control second state breaks (changes) in the first state.

The term "leading read" as used herein refers to a complete sequenced read that contains at least one k-mer that is a candidate breakpoint (variant).

Normally, in the case of heterozygous variation, only the reads derived from the altered allele contain the mutation or variant.

The term "block" refers to a leading read along with all reads derived from the sequencing of all four alleles involved (two coming from the first state genome and two coming from the second state genome) covering the same region as the leading read.

The term "similarity score" as used herein refers to numbers that help to identify how different sets of aligned sequences are, and can be used as part of the proposed method to measure the quality of an aligned block. Similarity scores can be vertical or horizontal. The former measures, for every position in a sequence, how many bases in the set of aligned sequences are different than the mode base. The latter measures, for every sequence in the set, how many positions of the sequence are different to the mode/consensus sequence. Similarity scores can be measured for different sets of sequences, e.g. the set of control sequences, the set of test sequences, or the set containing both.

The term "ambiguous path" as used herein refers to multiple possible sequence solutions in a given tree. It is referred here as the opposite of unique and unambiguous path or sequence.

The terms defined above are used in the following example for increasing their clarity and conciseness:
Imagine the read to be input:
A) (SEQ ID NO: 37)
   CACGGCAGCTGAGTCAACAGGTTCTTCCCAGGAGCGGACGGCGGTGGC CAGCAGGCACGTG (its length N=61).

After the quality filtering (step B) of the method), the next step of the method C) would be to generate a hashtable with all the k-mers of length X2. If X2 is taken to be 30, then, there should be N-X2+1 30-mers, that is, 61-30+1=32 (SEQ ID NO:38 - SEQ ID NO:69):

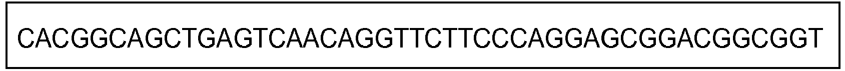

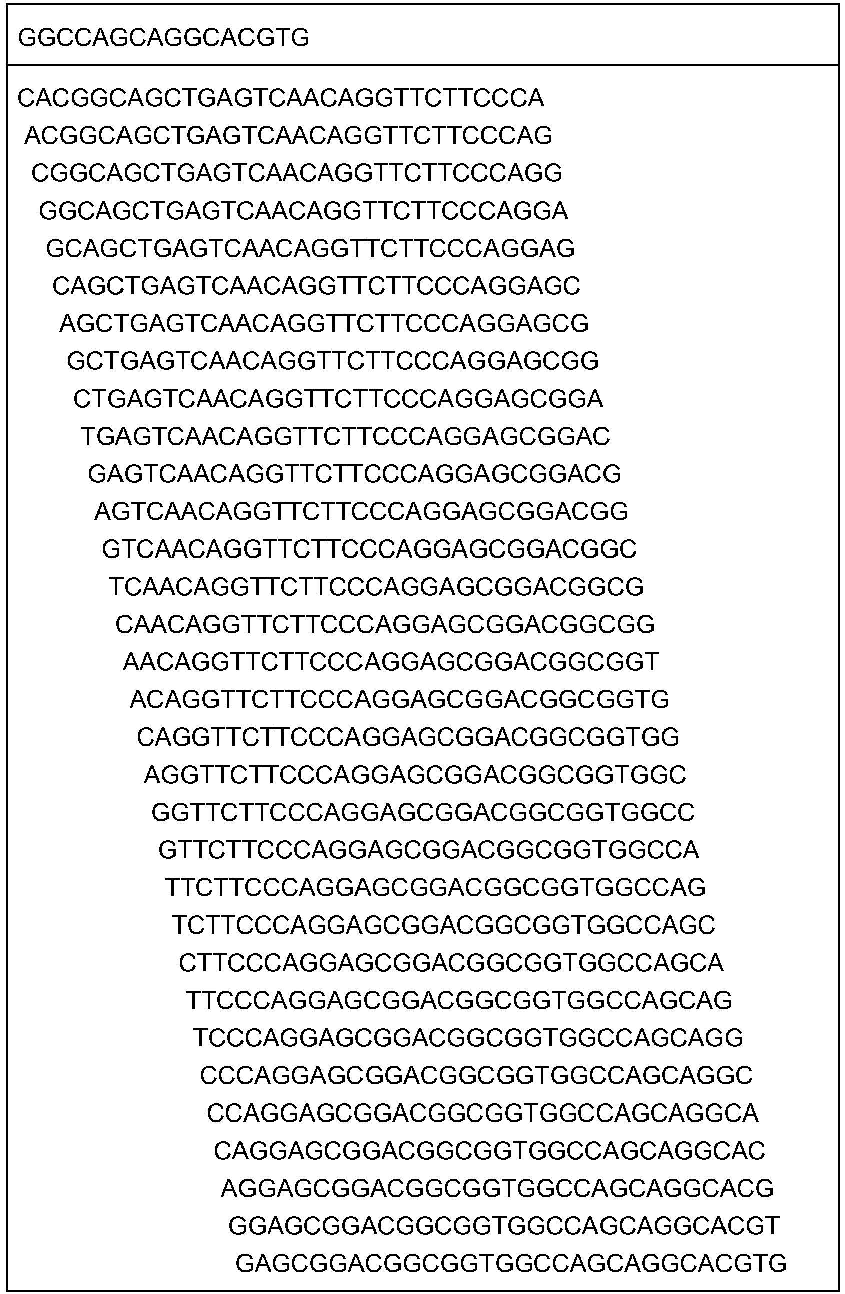

The hashtable to be generated with all k-mers and their number of times they are observed in first state and second state, would look like (SEQ ID NO:70 - SEQ ID NO:75):

| K-mer | Nor mal | Tumor |
|---|---|---|
| ACGGCAGCTGAGTCAACAGGTTCTTCCCAG | 0 | 1 |
| CGGCAGCTGAGTCAACAGGTTCTTCCCAGG | 0 | 1 |
| GGCAGCTGAGTCAACAGGTTCTTCCCAGGA | 0 | 1 |
| GCAGCTGAGTCAACAGGTTCTTCCCAGGAG | 0 | 1 |
| CAGCTGAGTCAACAGGTTCTTCCCAGGAGC | 0 | 1 |
| AGCTGAGTCAACAGGTTCTTCCCAGGAGCG | 0 | 1 |
| ... | | |

D) The next step in the method would be to detect variants between the first and second states. The first step would be to derive a stem for each one of the k-mers (SEQ ID NO:76 - SEQ ID NO:108):

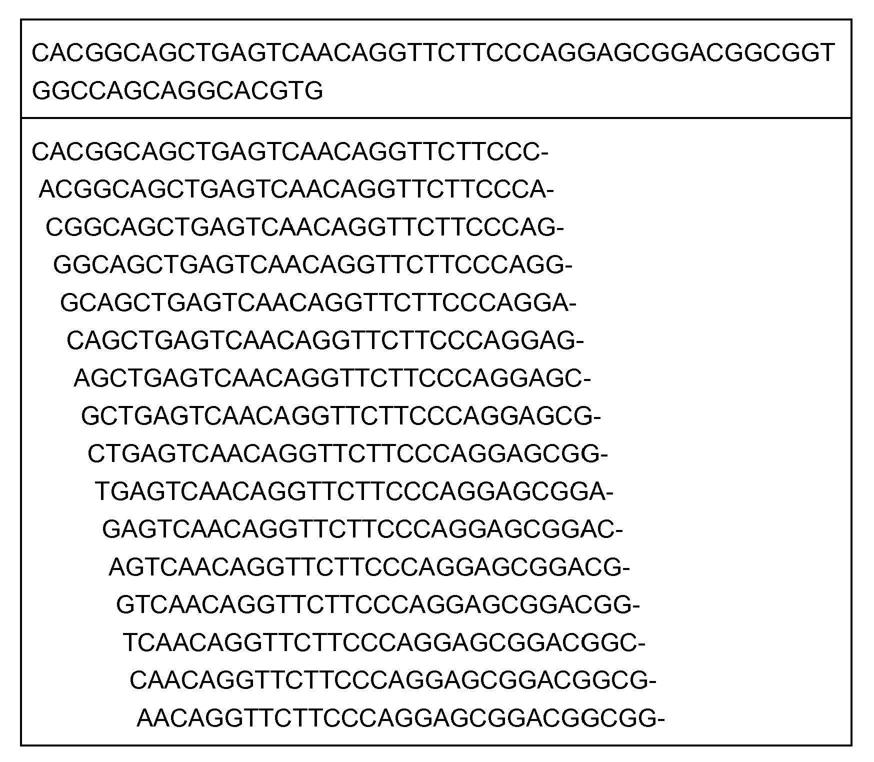

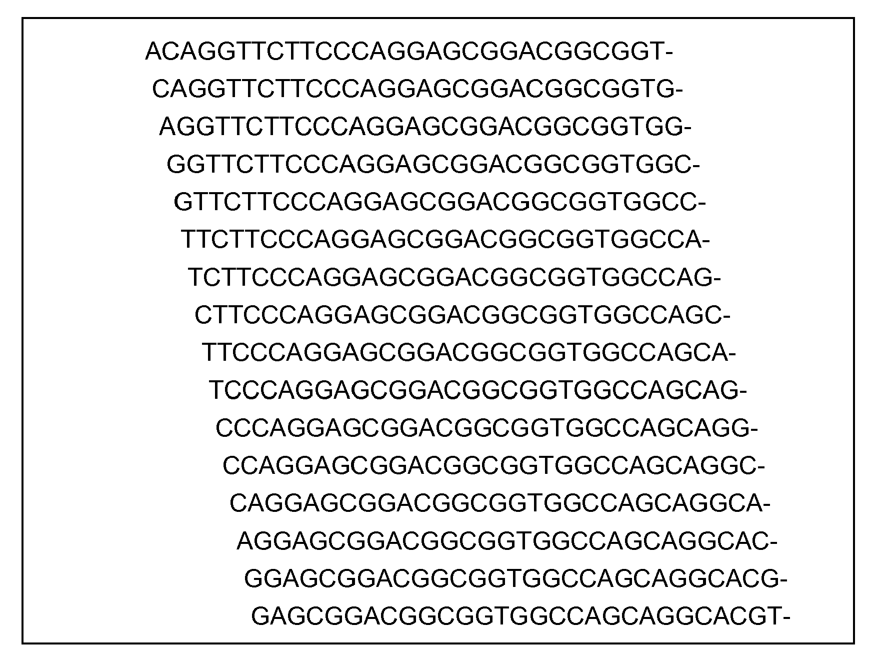

For each stem, inflections are to be generated. For instance, the second stem found in the table above would give the following 4 inflections (SEQ ID NO:109 - SEQ ID NO:113):

| | |
|---|---|
| | |
| | |
| | |
| | |

Find each one of the inflections in the hashtable:

| K-mer | Normal | Tumor |
|---|---|---|
| ACGGCAGCTGAGTCAACAGGTTCTTCCCAA | 0 | 1 |
| ACGGCAGCTGAGTCAACAGGTTCTTCCCAC | 0 | 4 |
| ACGGCAGCTGAGTCAACAGGTTCTTCCCAT | 0 | 1 |
| ACGGCAGCTGAGTCAACAGGTTCTTCCCAG | 0 | 1 |

If one inflection meets the requirements (has at least X3 reads with the same variation between the first and second states, and the amount of first state reads in second state reads is not over X4), select k-mer as candidate breakpoint:

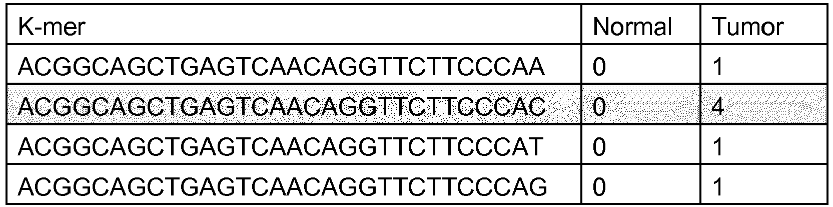

E) Clustering and filtering test and control reads derived from all candidate breakpoints:
For each candidate breakpoint selected in step D), retrieve reads which contain the stem of the k-mer (SEQ ID NO:114 - SEQ ID NO:116):

| Reads for candidate breakpoint based on k-mer |
|---|
| AGTCAACAGGTTCTTCCCAGGAGCGGACGC |
| |
| |

Then, for each read, we will end up with all k-mers that are candidate breakpoints, and their positions in the read (SEQ ID NO:117 - SEQ ID NO:126):

| Read #1 |
|---|
| |
| ACGGCAGCTGAGTCAACAGGTTCTTCCCAG (1) |
| GGCAGCTGAGTCAACAGGTTCTTCCCAGGA (3) |
| GCAGCTGAGTCAACAGGTTCTTCCCAGGAG (4) |
| CAGCTGAGTCAACAGGTTCTTCCCAGGAGC (5) |
| AGCTGAGTCAACAGGTTCTTCCCAGGAGCG (6) |
| GCTGAGTCAACAGGTTCTTCCCAGGAGCGG (7) |
| TGAGTCAACAGGTTCTTCCCAGGAGCGGAC (9) |
| GAGTCAACAGGTTCTTCCCAGGAGCGGACG (10) |
| AGTCAACAGGTTCTTCCCAGGAGCGGACGG (11) |

(SEQ ID NO:127 - SEQ ID NO:128)

| Read #2 |
|---|
| |
| AGTCAACAGGTTCTTCCCAGGAGCGGACGC (23) |

Reads with (X5) 7 k-mers containing candidate breakpoints within a window of (X6) 10 nucleotides are taken as leading reads (SEQ ID NO:129).

| Leading reads |
|---|
| |

Generate stems from leading reads (SEQ ID NO130 - SEQ ID NO:138):

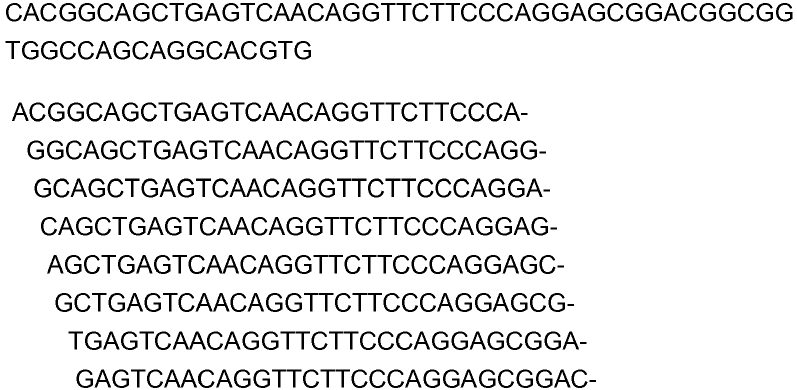

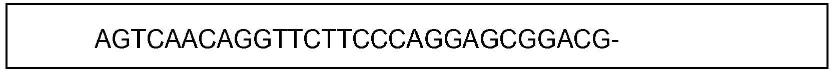

Build blocks by finding other candidate reads that share stems with leading reads (SEQ ID NO: 139 - SEQ ID NO:141):

| Leading read #1 |
|---|
| |

| Additional reads |
|---|
| |

| Shared stems |
|---|
| AGTCAACAGGTTCTTCCCAGGAGCGGACG- |

By executing the steps outlined above in the right order, the application of the computer-implemented method of the invention allows to easily cut the huge numbers of reads given in the input down to a much reduced number where most of the true positives are found, as will be seen in the experimental data found in the examples section (below).

As is revealed in Paszkiewicz K., et.al. "De novo assembly of short sequence reads" Brief Bioinform. 2010, vol. 11, pp. 475-472, the approximate minimum length that NGS reads must have in order to be able to reconstruct a genome is around 30. Bearing in mind the latter, a minimum length of approximately 30 bases was taken to be the minimum length of a productive read. 30 is a value that was found to be a viable cutoff for variable X2 in the definition of the method of the invention, although slightly smaller values might be viable as well.

As it has been cited above, the first aspect of the present invention is a computer-implemented method for identifying nucleic acid variants between two genomic states comprising the steps of: A) Inputting 2 sets of nucleic acid reads, which are sequences retrieved from a nucleotide sequencing method, wherein the first set of reads corresponds to cells representing a first test state, and the second set of reads corresponds to cells representing a second control state;B) Filtering the reads, wherein the filtering comprises: B1) Keeping only the reads with at least a percentage X1 of their bases with a Phred quality score higher than 20, being X1 equal to or above 90%; B2) Splitting the reads with an undefined nucleotide, giving one sequence before, and one sequence after the undefined nucleotide, the latter being discarded; and B3) Discarding the sequence reads with less than X2 bases, wherein X2 is from 25 to 50; C) Generating a hashtable structure comprising: C1) Generating a number of N-X2+1 new reads for each read of sequence length N, wherein the new N-X2+1 reads correspond to all k-mers with length X2 nucleotides; and C2) Building a hashtable structure, which comprises all the k-mers generated in step C1) and further comprises the number of times each k-mer is observed in the two sets of reads corresponding to first and second states.D) Detecting candidate variants in the sequence between first state and second state, wherein a k-mer of the hashtable structure is taken as a candidate breakpoint, which represents a variant between the first and second states, if it fulfills all the following requirements: D1) At least one inflection based on a k-mer's stem must have at least X3 reads with the same variation between first and second states, being X3 at least 2; 2) The percentage of first state reads in second state reads is not over a threshold X4, to account for possible contamination of control state reads with test state reads, wherein X4 is at least 5%. E) Clustering and filtering test and control reads derived from all candidate breakpoints accepted in step D to build blocks, by carrying out the steps: E1) Retrieving reads which contain the stem of at least one k-mer that represents the candidate breakpoint selected in step D); E2) The reads of step E1) with at least X5 k-mer variants within a window of X6 nucleotides are taken as leading reads, wherein X5 is at least 7 and X6 is at least 10; E3) Reads whose k-mers share at least one stem with a leading read are merged to give a block; and E4) If the nucleic acid whose variant is being identified is a double stranded DNA, then both forward and reverse variants are taken into account when building the block. F) Aligning blocks taking their leading reads as a reference: F1) For each read in the block, take the leading read's stem and find the longest inflection or partial inflection between the read and the leading read. F2) Successively position each read so that its matching inflection or partial inflection is aligned against the leading read.

In a particular embodiment of the first aspect of the invention, the computer-implemented method further comprises the step following F2: F3) F3) Obtaining first state scores, second state scores, and global similarity scores of each position in the block by measuring a ratio of most frequent nucleotide in that position relative to the total number of nucleotides.

In a particular embodiment of the first aspect of the invention, the computer-implemented method further comprises the step: G) Cataloguing and annotating blocks according to the following: G1) If blocks between the first and second states only differ in one substituted nucleotide, the variant is catalogued as containing a single nucleotide variant and the single nucleotide variant is annotated; G2) If blocks between the first and second states differ in more than one nucleotide but the whole difference in sequence is contained within the block, the variant is catalogued as a small structural variant, and the small structural variant is annotated; and G3) If blocks between the first and second states differ in more than one nucleotide and the whole difference in sequence is not contained within the block, the variant is catalogued as a large structural variant, and the boundaries of all large structural variants are extended by retrieving blocks overlapping at least X2 nucleotides in an iterative process which ends when the extended sequence reaches 200 nucleotides or when an ambiguous path is found.

In a particular embodiment of the first aspect of the invention, the computer-implemented method further comprises the step: H) Filtering of the blocks, according to the following: H1) The percentage of second state reads in first state reads is not over a threshold X7, to account for possible contamination of test state reads with control state reads, wherein X7 is at least 20%;

In a particular embodiment of the first aspect of the invention, the method further comprises optionally mapping second state blocks, and subsequently mapping first state blocks, on a reference genome.

In a particular embodiment of the first aspect of the invention, optionally in combination with any embodiment above or below, X1 is equal or above 95%.

In a particular embodiment of the first aspect of the invention, optionally in combination with any embodiment above or below, X1 is equal or above 99%.

In a particular embodiment of the first aspect of the invention, optionally in combination with any embodiment above or below, X2 is from 25 to 40.

In a particular embodiment of the first aspect of the invention, optionally in combination with any embodiment above or below, X2 is from 30 to 35.

In a particular embodiment of the first aspect of the invention, optionally in combination with any embodiment above or below, X2 is from 30 to 32.

In a particular embodiment of the first aspect of the invention, optionally in combination with any embodiment above or below, X2 is equal to 30.

In a particular embodiment of the first aspect of the invention, optionally in combination with any embodiment above or below, X3 is equal or above 4.

In a particular embodiment of the first aspect of the invention, optionally in combination with any embodiment above or below, X3 is equal or above 6.

In a particular embodiment of the first aspect of the invention, optionally in combination with any embodiment above or below, X3 is equal or above 8.

In a particular embodiment of the first aspect of the invention, X3 is directly proportional to the depth of coverage in the sequencing experiment. This means that, the deeper the coverage, the more restrictive (higher) is the value for X3.

In a particular embodiment of the first aspect of the invention, optionally in combination with any embodiment above or below, X4 is between 5-10%.

In a particular embodiment of the first aspect of the invention, optionally in combination with any embodiment above or below, X4 is between 5-7%.

In a particular embodiment of the first aspect of the invention, optionally in combination with any embodiment above or below, X4 is 5%.

X4 is expressed as a percentage, reflecting the maximum accepted ratio of first state (test) reads vs. second state (control) reads for each of the k-mers, and represents the levels of contamination expected for each of the samples (usually in the direction of tumor cells within normal samples). This value should be set by the user accordingly. Setting up a low value for X4 ensures high specificity but might result in a lower sensitivity, whereas the selection of high values, might result in the accumulation of false positives.

In a particular embodiment of the first aspect of the invention, optionally in combination with any embodiment above or below, X5 is from 10 to 15.

In a particular embodiment of the first aspect of the invention, optionally in combination with any embodiment above or below, X5 is from 12 to 14.

In a particular embodiment of the first aspect of the invention, optionally in combination with any embodiment above or below, X6 is from 12 to 25.

In a particular embodiment of the first aspect of the invention, optionally in combination with any embodiment above or below, X6 is from 12 to 20.

In a particular embodiment of the first aspect of the invention, optionally in combination with any embodiment above or below, X6 is from 12 to 15.

In a particular embodiment of the first aspect of the invention, optionally in combination with any embodiment above or below, X7 is between 20-25%.

In a particular embodiment of the first aspect of the invention, optionally in combination with any embodiment above or below, X7 is 20%.

In a particular embodiment of the first aspect of the invention, optionally in combination with any embodiment above or below, X5 is from 10 to 15 and the threshold X6 is from 12 to 20.

In a particular embodiment of the first aspect of the invention, optionally in combination with any embodiment above or below, X5 is from 12 to 14 and the threshold X6 is from 12 to 15.

In a particular embodiment of the first aspect of the invention, the first set of reads corresponds to pathological cells of a patient, and the second set of reads corresponds to non-pathological cells of the same patient;
In another particular embodiment of the first aspect of the invention, the first set of reads corresponds to cancer cells of a patient, and the second set of reads corresponds to non-cancer cells of the same patient.

In another particular embodiment of the first aspect of the invention, the first set of reads corresponds to virus-infected cells of a patient, and the second set of reads corresponds to non-infected cells of the same patient.

In another particular embodiment of the first aspect of the invention, the first set of reads and the second set of reads correspond to the same cell of the same patient in two different developmental stages.

In another particular embodiment of the first aspect of the invention, the first set of reads corresponds to cells of a patient which have been exposed to a drug, and the second set of reads corresponds to cells of the same patient which have not been exposed to a drug.

In a particular embodiment of the first aspect of the invention, the first set of reads corresponds to cells of a tissue, and the second set of reads corresponds to cells of another tissue of the same or a different individual.

Although the present invention has been described in detail for purpose of illustration, it is understood that such detail is solely for that purpose, and variations can be made therein by those skilled in the art without departing from the scope of the invention.

Thus, while the preferred embodiments of the methods and of the systems have been described in reference to the environment in which they were developed, they are merely illustrative of the principles of the invention. Other embodiments and configurations may be devised without departing from the scope of the appended claims.

Further, although the embodiments of the invention comprise processes performed in computer systems, the invention also extends to computer systems and to computer programs (which may be embodied on a storage medium and/or carried on a carrier signal) adapted for putting the invention into practice.

Accordingly, the invention also provides a computer program product comprising program instructions for causing a computer system to perform the method for identifying nucleic acid variants between two genomic states as defined above.

In a preferred embodiment, the computer program product is embodied on a storage medium.

In another preferred embodiment, the computer program product is carried on a carrier signal. The carrier may be any entity or device capable of carrying the program.

As it has been cited above, a fourth aspect of the invention is a computer system comprising a processor and a memory, wherein the memory stores computer executable instructions that, when executed by the processor, cause the system to perform the method for identifying nucleic acid variants between two genomic states.

In a preferred embodiment of the fourth aspect of the invention, the system may further comprise a hardware accelerator, which may be in some examples an FPGA or a GPU.

Throughout the description and claims the word "comprise" and variations of the word, are not intended to exclude other technical features, additives, components, or steps. Furthermore, the word "comprise" and its variations encompasses the term "consisting of". Additional objects, advantages and features of the invention will become apparent to those skilled in the art upon examination of the description or may be learned by practice of the invention. The following examples are provided by way of illustration, and they are not intended to be limiting of the present invention. Furthermore, the present invention covers all possible combinations of particular and preferred embodiments described herein.

### EXAMPLES

Examples of using the method of the invention for detecting characterizing sequence variants in nucleic acid sequences are given below.

In the in silico tests it is revealed that the method of the invention is capable of identifying SNVS and SVs of all sorts and sizes. Remarkably, the method of the invention is even proven to be capable of identifying novel non-human insertions. In one of the examples found below, the method is remarkably capable of detecting the insertion of a virus where, other methods (including the one disclosed in Moncunill et al., *ibid*) fail.

### Material and methods

### An implementation of the computer-implemented method

The general structure and the complete variant identification and characterization carried out by the method of the invention comprise the steps outlined below:

### A) Input data.

As input, the method takes high quality sequences data directly from FASTQ files of tumor and non-tumor control cells samples of the same individual. Alternatively, it is also able to accept BAM files, from which it extracts all the sequencing reads. Tumor sample corresponds to the first state and non-tumor control sample correspond to the second state.

### B) Filtering the data.

When inputting the data, the user can define a cut-off so that reads having over a certain threshold of their bases with a Phred quality score <q20 are discarded. X1=90 has been found to be especially suited for the purposes tested. This means that only reads with at least 90% of their bases with a Phred quality score higher than 20 are kept. In the case of the presence of undefined base pairs ("N"), these are removed and the original sequence is split forming new shorter reads, which are considered only if they are longer than X2 base pairs.

In order to lower the amount of space needed to store k-mers, they are converted into integers by mapping each base of the k-mer to a 2-bit code. For instance, a k-mer of length 32 represented as a sequence of characters takes 256 bits, but after the conversion it is turned into a single value of 64 bits.

In a particular embodiment of the computer-implemented method, wherein hardware accelerator(s) may be used,, reads that don't meet the aforementioned quality criteria are discarded by means of marking their k-mers as discardable, which are then ignored in the subsequent steps of the pipeline. Marking k-mers as discardable instead of deleting them immediately enables a faster pipeline without conditional execution.

### C) Generating a hashtable structure.

After the quality filtering step of the method, the next step of the method is to generate a hashtable with all the k-mers of length X2 using all high quality tumor and non-tumor control reads (see Table 1 below for a simplified version). If X2 is taken to be 28, then, there should be N-X2+1, that is, 100-28+1=73 resultant k-mers for a 100-nucleotide read. Each of k-mers generated is inserted into the hashtable and their number of times they are observed in tumor (test state) and non-tumor (second control state) cells.

The mapping of k-mers to their observed frequencies in the input is, generally speaking, one to one, meaning each entry of the associative data structure contains a pair of frequencies. In order to find the position where to store data into an associative structure a hash function is used to compute an index into a position, from which the desired value can be stored and retrieved. Any function that guarantees a homogeneous distribution of the results can be used as hash function.

**Table 1 (SEQ ID NO:142 - SEQ ID NO:145)**

| kmer (length X2) | Frequencies |
|---|---|
| ACTGACTGACTGACTGACTGACTGACTGAA | (0,1) |
| ACTGACTGACTGACTGACTGACTGACTGAC | (1,0) |
| ACTGACTGACTGACTGACTGACTGACTGAG | (23,2) |
| ACTGACTGACTGACTGACTGACTGACTGAT | (9,10) |
| ... | |

Each item of the hashtable consist of its k-mer (in nucleotide string or encoded key format), along with its pair frequencies in the first state and second state sets of reads (Table 1).

In particular embodiments of this aspect, each entry of the associative data structure may contain frequencies for more than one k-mer. This is accomplished by means of indexing stems instead of k-mers. Table 2 below

| stem (length X2-1) | Frequencies |
|---|---|
| ACTGACTGACTGACTGACTGACTGACTGA | A: (0,1) C: (1,0) G: (23,2) T: (9,10) |
| ACTGACTGACTGACTGACTGACTGACTGC | |
| ... | |

depicts such an example, where stems are basically the original k-mer truncating the last base, which is then included as part of the list of frequencies. Indexing stems instead of k-mers improves the locality of the data, reducing the number of lookup queries.

### Table 2 (SEQ ID NO:146 and SEQ ID NO:147)

In a particular embodiment, the hash function operates over the encoded key as described in step B instead of the k-mer containing a string of nucleotides. In order to lower the number of updates to the associative data structure, a particular embodiment of this aspect involves generating an additional structure to store the set of k-mers seen only once, meaning the main data structure is only updated for k-mers with a frequency of 2 or more.

In another embodiment, a lower number of updates to the main data structure is achieved by generating partial data structures containing frequencies for a subset of the input, which are then merged into the main associative data structure.

D) Detecting k-mers containing variants once all the reads are derived on k-mers and loaded into the hashtable structure, the next step consists in identifying all tumor specific reads. Inventors expect that variants generate new and distinct sequences in the tumor genome compared to the non-mutated control genome.

If one inflection meets the requirements: has at least 4 (X3) k-mers with the same variation between tumor cells and maximum 1 (X4) k-mer non-tumor control cells, then this k-mer is select as a candidate breakpoint.

The detection of entries of the hashtable containing k-mers with variants between the first and second state is accomplished by reading the filtered input again, and selecting all reads that contain k-mers whose frequencies meet certain criteria.

In addition to selecting candidate variants, in this step the implementation also selects additional information needed during later steps of the pipeline, namely: 1) selection of relative reads; 2) position of candidate k-mers for each read; and 3) map of k-mers to reads. In particular, the selection of relative reads is done based on stems, and checking whether any inflection matches the criteria described in the previous paragraph.

### E) Clustering and filtering test and control reads

For each candidate breakpoint selected in step D), retrieve reads which contain the stem of the k-mer. Then for each read, we will end up with all k-mers that are candidate breakpoints, and their positions in the read. Reads with 7 (X5) k-mers containing candidate breakpoints within a window of 10 (X6) nucleotides are taken as leading reads. This process is in order to find enough support information for each breakpoint detected (if not it is removed from the candidate list). Reads whose k-mers share at least one stem with a leading read are merged to give a block, and if the nucleic acid whose variant is being identified is a double stranded DNA, then both forward and reverse variants are taken into account when building the block.

### F) Aligning blocks taking their leading reads as a reference.

For each read in the block, inventors take the leading read's stem and find the longest inflection or partial inflection between the read and the leading read. Successively position each read so that its matching inflection or partial inflection is aligned against the leading read.

Ideally each block represents a region in the genome containing the mutated and the non-mutated version. In order to classify and characterize the type of variation identified, the method takes into account the align score for tumor block, non-tumor block and the sum up of both. Then the method extracts the consensus mutated and normal sequences from these blocks. The corresponding normal consensus sequence can be used at the end of the procedure and mapped onto a reference genome to obtain the coordinates of the variant.

### Optionally the method also can include step G

### G) Cataloguing and annotating blocks

Once all possible breakpoint blocks are defined, the next step consists in identifying and classifying the variation included there. At this point the method uses the aligning score to observe the differences in each group: tumor, non-tumor and both. For each position on the block supported by a read it puts a value depending on the similarity, so finally it has a representation of all the variability on the block. These aligning scores are recursively compared to identify differences between tumor and non-tumor samples. A first evaluation will search a consensus in non-tumor block in order to avoid false positives and wrong alignments from different regions, and the same way on tumor blocks. The next step searches for all the small variants, which consist on those that are completely included within the block (SNV and small SVs: insertions, deletions and inversions). All the blocks that do not match this criterion are then considered candidates for large SVs, i.e. likely to cover break points of intra or interchromosomal transitions, part of large deletions, insertions or inversions.

After small and large somatic variants are defined, the method of the invention identifies the coordinates of the changes by mapping onto a reference genome the normal consensus sequences corresponding to each of the variants, avoiding potential mapping conflicts derived from the presence of the variant, as usually happens when using reference-based approaches. Sequences mapping (with the same score) to several positions in the genome are discarded. The same process can be done by mapping onto virus databases to locate which are the viruses inserted in the genome being analyzed.

### Construction of the in silico chromosome 20

In order to measure and calibrate the detection capabilities (sensitivity) of the method of the invention, inventors executed it on a controlled system, consisting of modified sequences of the chromosome 20.

A personalized chromosome 20 was extracted from the hg19 reference genome downloaded from UCSC (with no repeat-masking) (http://www.ucsc.edu) and modified to match a randomly chosen human haplotype. This chromosome contains 148,639 variants consisting of 96,935 SNPs and 51,704 deletions. The catalogue of somatic variants further added to this personalized chromosome and constituting the target of the invention, includes random 168 SNVs, 12 random deletions, 18 random insertion, 6 inversions and 1 insertion of KI polyomavirus (extracted from: http://www.ncbi.n!m.nih.gov/Taxonomy/Browser/wwwtax.cgi?|v|=0&id=423445 )

In silico sequencing was simulated using ART Illumina (Huang W. et.al. "ART: a next-generation sequencing read simulator" Bioinformatics 2012, vol. 28, pp. 593-594). For this inventors, like on the previous in silico, first generated a profile using the M0004 sample to extract parameters, like sequencing variation or read length.

Analysis of the in silico chromosome 20 with the method of the invention

The method of the invention was run with using the next variables X1= 90, X2=28, X3=4, X4=1, X5=7 and X6=10 considered as default at the moment of the invention.
For the sake of clarity and simplicity, inventors have set up X4 as 1 in this example of the in silico chromosome 20.

### A System Configuration

The invention also comprised an efficient hardware configuration for the system focused on the execution of the computer program performing the method.
Due to the characteristics of the method, which involve a highly parallelizable input without dependencies and huge amounts of intermediate data, the input sources are split into chunks of C1 sequences and processed in a pipelined fashion. Thus, each step of the method is executed by a different component of the computer system, in parallel, over different chunks of input data. This strategy is more efficient in terms of execution time since it attempts to maximize the utilization of the system, and also overlaps data movement times.

When accelerators are capable of streaming data while computing, it is possible to add additional steps to the pipeline, eg. data is being sent to and received from the accelerators. When using accelerators that have interconnections unable to offer bidirectional concurrent transfers, data movement must be serialized and this could become the bottleneck of the entire pipeline. Generally, the bigger the number of sequences per chunk (C1), the higher the achievable bandwidth on data transfers from host system to accelerators and vice versa is. On the other hand, this number is limited by the memory capacity of the accelerators. Moreover, encoding the keys may require to sorting all keys and some parallel sorting algorithms require a number of items that's a power 2, meaning extra padding might be required in order to reach the next power of 2. When this happens, the number of reads sent to each accelerator must be accordingly chosen in order to minimize the number of pudding elements.

The hardware components of the computer system consisted of:

### A) Input Source

As input source, the processing pipeline reads high quality sequencing data from local disks or from network resources. An example of a network resource, is a sequencing machine connected to the system. In this case the system receives the input reads directly from the sequencing machine and starts elaborating at the same time as the sequencing machine is still working.

### B) Host System, and C) Accelerators

In order to compute and elaborate data, the invention comprises processors and accelerators for example, hardware in the form of PCI cards or network resources, for offloading part of the computation. The amount of work to offload to the accelerators depends on the interconnection between accelerator and host system as well on the processing power and memory of both processors and accelerators.
Ideally, processors will read the input sources that might need to be uncompressed and filtered in order to extract only sequence reads and quality markers. Once enough sequences to fill a chunk are loaded in memory, sequences and relative quality markers are sent to the accelerators. In turn, accelerators will: filter reads, convert all read fragments to their encoded key representation; reduce k-mers producing the <key, count> pairs; and eventually, hashing the keys to obtain the tuple <key, count, hash>. Once the data is transferred back to the system's main memory, processors will consume it updating the counters stored in the associative structure. Optionally, when accelerators have network interconnections and inputs are streamed from network resources, accelerators can read input directly from the source without requiring any work from the main system, offloading even more work. When the capabilities of the accelerators are limited, some of the processing steps can be carried out by the processors instead. For example, if transferring the tuple <key, count, hash> from accelerators to the host system becomes the bottleneck of the processing pipeline, it's wise to migrate the hashing step to the processors. In this way, it is reduced to 3/5 the amount of data that is transferred from the accelerators to the host system.

If the steps offloaded to the accelerators constitutes the bottleneck and having a higher number of updates to the hash table does not have an impact on the performance, the key conversion step can be disabled in order to increase the global throughput.

If more than one accelerator is available, data is split in different parts and each one of the parts is sent to a different accelerator. In order to efficiently offload the computation among multiple accelerators, the criterion used to split the data may take into account the available memory of each accelerator and may be as balanced as possible to the throughput offered by each accelerator. Which, for example, is: if two accelerators are available and one of them offers a throughput that is double of the other then the former accelerator should process an amount of data that is about 2/3 of the total, meanwhile the latter accelerator is about 1/3. If accelerators are different among each other and it's clear that different accelerators might suit better for different steps; then, the steps of the method are split among the available accelerator assigning each step to the accelerator that suits better. In this case, the output of one accelerator becomes the input of another and if no direct interconnection is available between the accelerators the host system must intervene to orchestrate data transfers. On the other hand, if no accelerators are available all the computation must be carried out by the main processors.

### D) Main Memory, and E) Memory Expansion Cards

In addition to staging intermediate data from a one step of the pipeline to the next one, main memory is also used to store a partial version of the associative structure. This in-memory structure is used to store k-mers seen just few times, while those seen more than I1 times are stored into a permanent associative structure in the memory expansions card.

In order to store the persistent associative structure containing all the useful information from normal (CNR) and tumoral (CTR) sample, memory expansion cards are used. When multiple cards are available inventors can use each card to store part of the associative structure allowing to split read requests among the cards increasing the possible number of in-flight requests proportionally with the number of the cards. Examples of an apparatus that can be used as memory expansion cards may be NVMe cards.

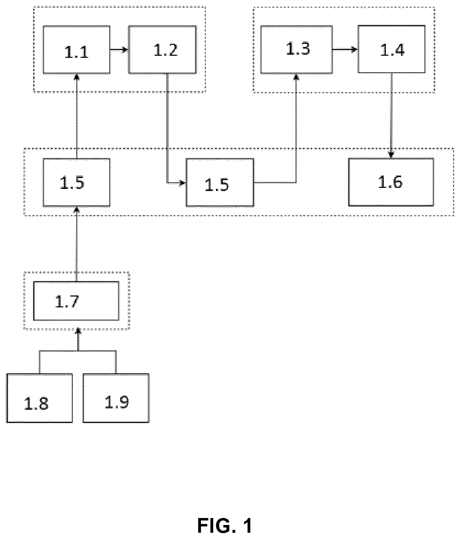

FIG. 1 shows the data flow from the input source, first to host system to load the reads, and then to accelerators for quality checking and base conversion. Afterwards, data is reduced again by the accelerators, which also generate the hash before data is loaded back into memory.

### Results

As explained above, to assess the performance of the method of the invention, inventors measured the fraction of somatic variants detected (sensitivity).

### In silico validation with chromosome 20

This sample was created exclusively in order to validate the method against an in silico sample with an insertion of a virus, also the sample includes different somatic mutations.
Inventors first observed that the calling of somatic SNVs and SVs is optimal with a sensitivity of 96.4% for SNVs, 96.2% for Small SVs, 100% for Large SVs and 100% for virus Insertion. It is remarkable the capacity of detecting large structural variants with such a high sensitivity (Table 3)

**Table 3. Assessment variant calling for chromosome 20**

| | Mutations | After filtering | % detection | After clustering | % detection |
|---|---|---|---|---|---|
| Point mutations | 168 | 167 | 99.4% | 162 | 96.4% |
| Small SVs (indels) | 26 | 26 | 100.0% | 25 | 96.2% |
| Large SVs | 10 | 10 | 100.0% | 10 | 100.0% |
| Virus | 1 | 1 | 100.0% | 1 | 100.0% |

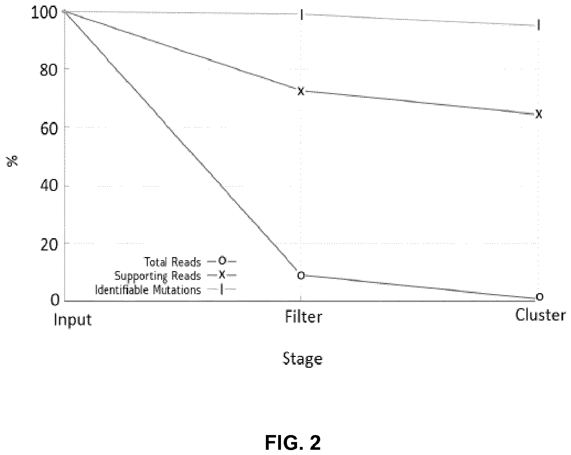

FIG. 2 provides an overview of the filtering capabilities of the invention, along with its sensitivity, at different stages of the execution. Step D) (filter) reduces the number of input reads to less than 10% of the input, while still keeping 99% of mutations identifiable, and high number of reads supporting those mutations (72%). Step E) (clustering) further decreases the size of relevant reads, while still keeping very high number of identifiable mutations.

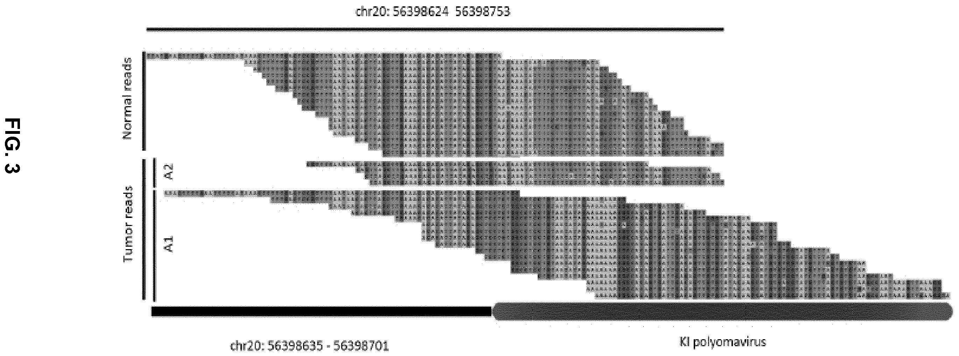

Besides that, it is also capable of detecting the insertion of the KI polyomavirus. The computer-implemented method of the invention allows finding viral integration events with better accuracy and recall than the available alternative methods which are based on pre-alignment steps of the reads onto a reference genome. (FIG. 3).

In the case of normal reads, inventors observed the genome sequence without any kind of structural variation. In contrast, on tumor reads (with heterozygosity A1-A2) inventors were able to detect the KI polyomavirus insertion on chromosome 20 at position 56398701.

In silico validation of the chromosome 20 insertion with the method disclosed in Moncunill et. al (ibid):
This method, with the default parameters, performed poorly in this test. Its results on large structural variants does not show any evidence of the detection of KI polyomavirus insertion on chromosome 20 at position 56398701, it was just able to describe variants from the own genome. Therefore, the method of the present invention was shown to be superior to the reference-free suffix-tree- based method described in Moncunill et al.

Taking into account all the results obtained at this point, the method of the invention has shown the capacity to detect all kinds of SNVs and SVs with great sensitivity without restrictions with the size. Also it is capable to detect the insertions of a virus in the genome with a base-pair resolution in comparison with the available alternative methods, which are based on suffix trees.

### REFERENCES CITED IN THE APPLICATION

Li, H., et.al. "Fast and accurate short read alignment with Burrows-Wheeler transform" Bioinformatics 2009, vol. 25, pp. 1754-1760
Medvedev P. "Computational methods for discovering structural variation with next-generation sequencing" Nat. Methods Suppl. 2009, vol. 6, S13-S20
Ding, L., et.al. "Expanding the computational toolbox for mining cancer genomes" Nat. Rev. Genet. 2014, vol. 15, pp. 556-570
   Chen K. "TIGRA: A targeted Iterative Graph Routing Assembler for Breakpoint Assembly" Genome Res. 2016, vol. 24, pp.310-317.
Zhuang, J. "Local sequence assembly reveals a high-resolution profile of somatic structural variations in 97 cancer genomes" Nucleic Acid Research 2015, vol. 43, pp.8146-8156
Moncunill V., et al., "Comprehensive characterization of complex structural variations by directly comparing genome sequence reads" Nature Biotech. 2014, vol. 32, pp. 1106-1112
Patro R., et. al. "Sailfish enables alignment-free isoform quantification from RNA-seq reads using lightweight algorithms" Nature Biotech. 2014, vol. 32, pp. 462-464
Nordstrom K.J.V, et.al. "Mutation identification by direct comparison of whole-genome sequencing data from mutant and wild-type individuals using k-mers" Nature Biotech. 2013, vol. 31, pp. 325-331
Paszkiewicz K., et.al. "De novo assembly of short sequence reads" Brief Bioinform. 2010, vol. 11, pp. 475-472
Huang W. et.al. "ART: a next-generation sequencing read simulator" Bioinformatics 2012, vol. 28, pp. 593-594

## Claims

1. A computer-implemented method for identifying of nucleic acid variants between two genomic states comprising the steps of:
A) Inputting 2 sets of nucleic acid reads, which are sequences retrieved from a nucleotide sequencing method, wherein the first set of reads corresponds to cells representing a first test state, and the second set of reads corresponds to cells representing a second control state;
B) Filtering the reads, wherein the filtering comprises:
B1) Keeping only the reads with at least a percentage X1 of their bases with a Phred quality score higher than 20, being X1 equal to or above 90%;
B2) Splitting the reads with an undefined nucleotide, giving one sequence before, and one sequence after the undefined nucleotide, the latter being discarded; and
B3) Discarding the sequence reads with less than X2 bases, wherein X2 is from 25 to 50;
C) Generating a hashtable structure comprising:
C1) Generating a number of N-X2+1 new reads for each read of sequence length N, wherein the new N-X2+1 reads correspond to all k-mers with length X2 nucleotides; and
C2) Building a hashtable structure, which comprises all the k-mers generated in step C1) and further comprises the number of times each k-mer is observed in the two sets of reads corresponding to first and second states.
D) Detecting candidate variants in the sequence between first state and second state, wherein a k-mer of the hashtable structure is taken as a candidate breakpoint, which represents a variant between the first and second states, if it fulfills all the following requirements:
D1) At least one inflection based on a k-mer's stem must have at least X3 reads with the same variation between first and second states, being X3 at least 2;
D2) The percentage of first state reads in second state reads is not over a threshold X4, to account for possible contamination of control state reads with test state reads, wherein X4 is at least 5%.
E) Clustering and filtering test and control reads derived from all candidate breakpoints accepted in step D to build blocks, by carrying out the steps:
E1) Retrieving reads which contain the stem of at least one k-mer that represents the candidate breakpoint selected in step D);
E2) The reads of step E1) with at least X5 k-mer variants within a window of X6 nucleotides are taken as leading reads, wherein X5 is at least 7 and X6 is at least 10;
E3) Reads whose k-mers share at least one stem with a leading read are merged to give a block; and
E4) If the nucleic acid whose variant is being identified is a double stranded DNA, then both forward and reverse variants are taken into account when building the block.
F) Aligning blocks taking their leading reads as a reference:
F1) For each read in the block, take the leading read's stem and find the longest inflection or partial inflection between the read and the leading read.
F2) Successively position each read so that its matching inflection or partial inflection is aligned against the leading read;
wherein:
"candidate breakpoint" means the candidate nucleotide position where the sequence changes, that is immediately flanking a sequence variant.
"inflection" means a fragment of length k that can be derived from extending a stem of a length lower than the length of a k-mer of length k; and
"stem" means a fragment of a k-mer of length k with S defined bases, where S < k, and k-S omitted (undefined) bases; a k-mer being understood as all possible substrings of length k that are contained in a string.

2. The computer-implemented method according to claim 1, further comprising the step following F2:
F3) Obtaining first state scores, second state scores, and global similarity scores of each position in the block by measuring a ratio of most frequent nucleotide in that position relative to the total number of nucleotides.

3. The computer-implemented method according to any one of claims 1-2, further comprising the step of:
G) Cataloguing and annotating blocks according to the following:
G1) If blocks between the first and second states only differ in one substituted nucleotide, the variant is catalogued as containing a single nucleotide variant and the single nucleotide variant is annotated;
G2) If blocks between the first and second states differ in more than one nucleotide but the whole difference in sequence is contained within the block, the variant is catalogued as a small structural variant, and the small structural variant is annotated; and
G3) If blocks between the first and second states differ in more than one nucleotide and the whole difference in sequence is not contained within the block, the variant is catalogued as a large structural variant, and the boundaries of all large structural variants are extended by retrieving blocks overlapping at least X2 nucleotides in an iterative process which ends when the extended sequence reaches 200 nucleotides or when an ambiguous path is found.

4. The computer-implemented method according to any one of the claims 1-3, further comprising the step of:
H) Filtering of the blocks, according to the following:
H1) The percentage of second state reads in first state reads is not over a threshold X7, to account for possible contamination of test state reads with control state reads, wherein X7 is at least 20%;

5. The computer-implemented method according to any of the claims 1 to 4 further comprising optionally mapping second state blocks, and subsequently mapping first state blocks, on a reference genome.

6. The computer-implemented method according to any one of claims 1 to 5, wherein X2 is from 25 to 40.

7. The computer-implemented method according to any one of claims 1 to 6, wherein X3 is equal to or above 3.

8. The computer-implemented method according to any one of claims 1 to 7, wherein threshold X4 is 5%.

9. The computer-implemented method according to any one of claims 1 to 8, wherein the threshold X5 is from 10 to 15 and the threshold X6 is from 12 to 20.

10. The computer-implemented method according to any one of claims 1 to 9, wherein the first set of reads corresponds to pathological cells of a patient and the second set of reads corresponds to non-pathological cells of the same patient.

11. A computer program product comprising program instructions for causing a computer system to perform the method for identifying nucleic acid variants between two genomic states as defined in any of claims 1 to 10.

12. The computer program product according to claim 11 embodied on a storage medium.

13. The computer program product according to claim 11 carried on a carrier signal.

14. A system for identifying nucleic acid variants between two genomic states comprising the steps of:
A) Computer/Electronic means for inputting 2 sets of nucleic acid reads, which are sequences retrieved from a nucleotide sequencing method, wherein the first set of reads corresponds to cells representing a first test state, and the second set of reads corresponds to cells representing a second control state;
B) Computer/Electronic means for filtering the reads, wherein the filtering comprises:
B1) Keeping only the reads with at least a percentage X1 of their bases with a Phred quality score higher than 20, being X1 equal to or above 90%;
B2) Splitting the reads with an undefined nucleotide, giving one sequence before, and one sequence after the undefined nucleotide, the latter being discarded; and
B3) Discarding the sequence reads with less than X2 bases, wherein X2 is from 25 to 50;
C) Computer/Electronic means for generating a hashtable structure comprising:
C1) Generating a number of N-X2+1 new reads for each read of sequence length N, wherein the new N-X2+1 reads correspond to all k-mers with length X2 nucleotides; and
C2) Building a hashtable structure, which comprises all the k-mers generated in step C1) and further comprises the number of times each k-mer is observed in the two sets of reads corresponding to first and second states.
D) Computer/Electronic means for detecting variants in the sequence between first state and second state, wherein a k-mer of the hashtable structure is taken as a candidate breakpoint, which represents a variant between the first and second states, if it fulfills all the following requirements:
D1) At least one inflection based on a k-mer's stem must have at least X3 reads with the same variation between first and second states, being X3 at least 2;
D2) The percentage of first state reads in second state reads is not over a threshold X4, to account for possible contamination of control state reads with test state reads, wherein X4 is at least 5%
E) Computer/Electronic means for clustering and filtering test and control reads derived from all candidate breakpoints accepted in step D to build blocks, by carrying out the steps:
E1) Retrieving reads which contain the stem of at least one k-mer that represents the candidate breakpoint selected in step D);
E2) The reads of step E1) with at least X5 k-mer variants within a window of X6 nucleotides are taken as leading reads, wherein X5 is at least 7 and X6 is at least 10;
E3) Reads whose k-mers share at least one stem with a leading read are merged to give a block; and
E4) If the nucleic acid whose variant is being identified is a double stranded DNA, then both forward and reverse variants are taken into account when building the block.
F) Computer/Electronic means for aligning blocks taking their leading reads as a reference:
F1) For each read in the block, take the leading read's stem and find the longest inflection or partial inflection between the read and the leading read.
F2) Successively position each read so that its matching inflection or partial inflection is aligned against the leading read,
wherein:
"candidate breakpoint" means the candidate nucleotide position where the sequence changes, that is immediately flanking a sequence variant.
"inflection" means a fragment of length k that can be derived from extending a stem of a length lower than the length of a k-mer of length k; and
"stem" means a fragment of a k-mer of length k with S defined bases, where S < k, and k-S omitted (undefined) bases; a k-mer being understood as all possible substrings of length k that are contained in a string.

15. A computer system comprising a processor and a memory, wherein the memory stores computer executable instructions that, when executed by the processor, cause the system to perform the method for identifying nucleic acid variants between two genomic states as defined by any of claims 1 to 10.

## Patentansprüche

1. Computerimplementiertes Verfahren zur Identifizierung von Nukleinsäurevarianten zwischen zwei genomischen Zuständen, umfassend die folgenden Schritte:
A) Eingabe von 2 Sätzen von Nukleinsäureablesungen, bei denen es sich um Sequenzen handelt, die aus einem Nukleotidsequenzierungsverfahren gewonnen wurden, wobei der erste Satz von Ablesungen Zellen entspricht, die einen ersten Testzustand darstellen, und der zweite Satz von Ablesungen Zellen entspricht, die einen zweiten Kontrollzustand darstellen;
B) Filtern der Ablesungen, wobei das Filtern umfasst:
B1) Beibehaltung nur der Ablesungen mit mindestens einem Prozentsatz X1 ihrer Basen mit einem Phred-Qualitätswert über 20, wobei X1 gleich oder höher als 90 % ist;
B2) Aufspaltung der Ablesungen mit einem undefinierten Nukleotid, wobei eine Sequenz vor und eine Sequenz nach dem undefinierten Nukleotid angegeben wird, wobei die letztere ausgelassen wird; und
B3) Das Auslassen der Sequenzablesungen mit weniger als X2 Basen, wobei X2 zwischen 25 und 50 liegt;
C) Erstellen einer Hashtabellenstruktur umfassend:
C1) Erstellen einer Anzahl von N-X2+1 Neuablesungen für jede Ablesung der Sequenzlänge N, wobei die neuen N-X2+1 Ablesungen allen k-Meren mit Nukleotiden der Länge X2 entsprechen; und
C2) Aufbau einer Hashtabellenstruktur, die alle in Schritt C1) erzeugten k-mers umfasst und ferner die Anzahl der Beobachtungen jedes k-mers in den beiden Sätzen von Ablesungen umfasst, die dem ersten und zweiten Zustand entsprechen.
D) Erfassen von Kandidatenvarianten in der Sequenz zwischen dem ersten Zustand und dem zweiten Zustand, wobei ein k-mer der Hashtabellenstruktur als Kandidatenbruchpunkt genommen wird, der eine Variante zwischen dem ersten und dem zweiten Zustand darstellt, wenn er alle folgenden Anforderungen erfüllt:
D1) Mindestens eine Beugung, die auf einem k-mer-Stamm basiert, muss mindestens X3 Ablesungen mit der gleichen Abweichung zwischen dem ersten und zweiten Zustand aufweisen, wobei X3 mindestens 2 ist;
D2) Der Prozentsatz der Ablesungen des ersten Zustands in den Ablesungen des zweiten Zustands liegt nicht über einem Schwellenwert X4, um eine mögliche Kontamination der Ablesungen des Kontrollzustands mit den Ablesungen des Testzustands zu berücksichtigen, wobei X4 mindestens 5 % beträgt.
E) Clustern und Filtern von Test- und Kontrollablesungen, die von allen in Schritt D akzeptierten Kandidatenbruchpunkten abgeleitet werden, um Blöcke zu bilden, durch Ausführen der Schritte:
E1) Abrufen von Ablesungen, die den Stamm von mindestens einem k-mer enthalten, der den in Schritt D) ausgewählten Kandidatenbruchpunkt darstellt;
E2) Die Ablesungen von Schritt E1) mit mindestens X5 k-mer-Varianten innerhalb eines Fensters von X6 Nukleotiden werden als Leitablesungen genommen, wobei X5 mindestens 7 ist und X6 mindestens 10 ist;
E3) Ablesungen, deren k-mers mindestens einen Stamm mit einer Leitablesung teilen, werden zu einem Block zusammengeführt; und
E4) Handelt es sich bei der Nukleinsäure, deren Variante identifiziert wird, um eine doppelsträngige DNS, dann werden beim Blockaufbau sowohl Vorwärts- als auch Rückwärtsvarianten berücksichtigt.
F) Das Ausrichten von Blöcken unter Berücksichtigung ihrer Leitablesung als Referenz:
F1) Nehmen Sie für jede Ablesung im Block den Stamm der Leitablesung und suchen Sie die längste Beugung oder Teilbeugung zwischen der Ablesung und der Leitablesung.
F2) Positionieren Sie jede Ablesung nacheinander so, dass ihre übereinstimmende Beugung oder Teilbeugung an der Leitablesung ausgerichtet ist;
wobei:
"Kandidatenbruchpunkt" die Position des Nukleotidkandidaten bezeichnet, an der sich die Sequenz ändert, d.h. die unmittelbar eine Sequenzvariante flankiert.
"Beugung" ein Fragment der Länge k bezeichnet, das aus der Verlängerung eines Stammes mit einer Länge kleiner als die Länge eines k-mer der Länge k abgeleitet werden kann; und
"Stamm" ist ein Fragment eines k-mer der Länge k mit S definierten Basen, wobei S < k, und k-S ausgelassene (undefinierte) Basen sind; unter einem k-mer versteht man alle möglichen Teilzeichenketten der Länge k, die in einer Zeichenkette enthalten sind.

2. Computerimplementiertes Verfahren nach Anspruch 1, umfassend ferner den auf F2 folgenden Schritt:
F3) Ermittlung von Erstzustandsbewertungen, Zweitzustandsbewertungen und globalen Ähnlichkeitsbewertungen für jede Position im Block durch Messung des Verhältnisses der häufigsten Nukleotide an dieser Position im Verhältnis zur Gesamtzahl der Nukleotide.

3. Computerimplementiertes Verfahren nach einem der Ansprüche 1-2, umfassend ferner den Schritt:
G) Katalogisierung und Annotation von Blöcken wie folgt:
G1) Unterscheiden sich Blöcke zwischen dem ersten und dem zweiten Zustand nur in einem substituierten Nukleotid, wird die Variante als eine einzige Nukleotidvariante katalogisiert und die einzige Nukleotidvariante annotiert;
G2) Unterscheiden sich Blöcke zwischen dem ersten und dem zweiten Zustand in mehr als einem Nukleotid, aber der gesamte Unterschied in der Sequenz ist innerhalb des Blocks enthalten, wird die Variante als kleine Strukturvariante katalogisiert, und die kleine Strukturvariante wird annotiert; und
G3) Unterscheiden sich Blöcke zwischen dem ersten und zweiten Zustand in mehr als einem Nukleotid und ist der gesamte Unterschied in der Sequenz nicht im Block enthalten, wird die Variante als große Strukturvariante katalogisiert, und die Abgrenzungen aller großen Strukturvarianten werden erweitert, indem Blöcke, die mindestens X2 Nukleotide überlappen, in einem iterativen Prozess abgerufen werden, der endet, wenn die erweiterte Sequenz 200 Nukleotide erreicht oder wenn ein mehrdeutiger Pfad gefunden wird.

4. Computerimplementiertes Verfahren nach einem der Ansprüche 1-3, umfassend ferner den Schritt:
G) Filtern der Blöcke wie folgt:
H1) Der Prozentsatz der Ablesungen des zweiten Zustands in den Ablesungen des ersten Zustands liegt nicht über einem Schwellenwert X7, um eine mögliche Kontamination der Ablesungen des Testszustands mit den Ablesungen des Kontrollzustands zu berücksichtigen, wobei X7 mindestens 20 % beträgt;

5. Computerimplementiertes Verfahren nach einem der Ansprüche 1 bis 4, umfassend ferner die optionale Kartierung zweiter Zustandsblöcke und die anschließende Kartierung erster Zustandsblöcke auf einem Referenzgenom.

6. Computerimplementiertes Verfahren nach einem der Ansprüche 1 bis 5, wobei X2 zwischen 25 und 40 liegt.

7. Computerimplementiertes Verfahren nach einem der Ansprüche 1 bis 6, wobei X3 gleich oder höher 3 ist.

8. Computerimplementiertes Verfahren nach einem der Ansprüche 1 bis 7, wobei der Schwellenwert X4 5% beträgt.

9. Computerimplementiertes Verfahren nach einem der Ansprüche 1 bis 8, wobei der Schwellenwert X5 zwischen 10 und 15 und der Schwellenwert X6 zwischen 12 und 20 liegt.

10. Computerimplementiertes Verfahren nach einem der Ansprüche 1 bis 9, wobei der erste Satz von Ablesungen pathologischen Zellen eines Patienten entspricht und der zweite Satz von Ablesungen nicht-pathologischen Zellen desselben Patienten entspricht.

11. Computerprogrammprodukt, umfassend Programmbefehle, um ein Computersystem zu veranlassen, das Verfahren zur Identifizierung von Nukleinsäurevarianten zwischen zwei genomischen Zuständen, wie in einem der Ansprüche 1 bis 10 definiert, durchzuführen.

12. Computerprogrammprodukt nach Anspruch 11, ausgeführt auf einem Speichermedium.

13. Computerprogrammprodukt nach Anspruch 11, getragen auf einem Trägersignal.

14. System zur Identifizierung von Nukleinsäurevarianten zwischen zwei genomischen Zuständen, umfassend die folgenden Schritte:
A) Computer/Elektronische Vorrichtung zur Eingabe von 2 Sätzen von Nukleinsäureablesungen, bei denen es sich um Sequenzen handelt, die aus einem Nukleotidsequenzierungsverfahren gewonnen wurden, wobei der erste Satz von Ablesungen Zellen entspricht, die einen ersten Testzustand darstellen, und der zweite Satz von Ablesungen Zellen entspricht, die einen zweiten Kontrollzustand darstellen;
B) Computer/Elektronische Vorrichtung zum Filtern der Ablesungen, wobei das Filtern umfasst:
B1) Beibehaltung nur der Ablesungen mit mindestens einem Prozentsatz X1 ihrer Basen mit einem Phred-Qualitätswert über 20, wobei X1 gleich oder höher als 90 % ist;
B2) Aufspaltung der Ablesungen mit einem undefinierten Nukleotid, wobei eine Sequenz vor und eine Sequenz nach dem undefinierten Nukleotid angegeben wird, wobei die letztere ausgelassen wird; und
B3) Das Auslassen der Sequenzablesungen mit weniger als X2 Basen, wobei X2 zwischen 25 und 50 liegt;
C) Computer/Elektronische Vorrichtung zur Erzeugung einer Hashtable-Struktur, bestehend aus:
C1) Erstellen einer Anzahl von N-X2+1 Neuablesungen für jede Ablesung der Sequenzlänge N, wobei die neuen N-X2+1 Ablesungen allen k-Meren mit Nukleotiden der Länge X2 entsprechen; und
C2) Aufbau einer Hashtabellenstruktur, die alle in Schritt C1) erzeugten k-mers umfasst und ferner die Anzahl der Beobachtungen jedes k-mers in den beiden Sätzen von Ablesungen umfasst, die dem ersten und zweiten Zustand entsprechen.
D) Computer Elektronische Vorrichtung zum Erfassen von Kandidatenvarianten in der Sequenz zwischen dem ersten Zustand und dem zweiten Zustand, wobei ein k-mer der Hashtabellenstruktur als Kandidatenbruchpunkt genommen wird, der eine Variante zwischen dem ersten und dem zweiten Zustand darstellt, wenn er alle folgenden Anforderungen erfüllt:
D1) Mindestens eine Beugung, die auf einem k-mer-Stamm basiert, muss mindestens X3 Ablesungen mit der gleichen Abweichung zwischen dem ersten und zweiten Zustand aufweisen, wobei X3 mindestens 2 ist;
D2) Der Prozentsatz der Ablesungen des ersten Zustands in den Ablesungen des zweiten Zustands liegt nicht über einem Schwellenwert X4, um eine mögliche Kontamination der Ablesungen des Kontrollzustands mit den Ablesungen des Testzustands zu berücksichtigen, wobei X4 mindestens 5 % beträgt
E) Computer/Elektronische Vorrichtung zum Clustern und Filtern von Test- und Kontrollablesungen, die von allen in Schritt D akzeptierten Kandidatenbruchpunkten abgeleitet werden, um Blöcke zu bilden, durch Ausführen der Schritte:
E1) Abrufen von Ablesungen, die den Stamm von mindestens einem k-mer enthalten, der den in Schritt D) ausgewählten Kandidatenbruchpunkt darstellt;
E2) Die Ablesungen von Schritt E1) mit mindestens X5 k-mer-Varianten innerhalb eines Fensters von X6 Nukleotiden werden als Leitablesungen genommen, wobei X5 mindestens 7 ist und X6 mindestens 10 ist;
E3) Ablesungen, deren k-mers mindestens einen Stamm mit einer Leitablesung teilen, werden zu einem Block zusammengeführt; und
E4) Handelt es sich bei der Nukleinsäure, deren Variante identifiziert wird, um eine doppelsträngige DNS, dann werden beim Blockaufbau sowohl Vorwärts- als auch Rückwärtsvarianten berücksichtigt.
F) Computer/Elektronische Vorrichtung zum Ausrichten von Blöcken unter Berücksichtigung ihrer Leitablesung als Referenz:
F1) Nehmen Sie für jede Ablesung im Block den Stamm der Leitablesung und suchen Sie die längste Beugung oder Teilbeugung zwischen der Ablesung und der Leitablesung.
F2) Positionieren Sie jede Ablesung nacheinander so, dass ihre übereinstimmende Beugung oder Teilbeugung an der Leitablesung ausgerichtet ist, wobei:
"Kandidatenbruchpunkt" die Position des Nukleotidkandidaten bezeichnet, an der sich die Sequenz ändert, d.h. die unmittelbar eine Sequenzvariante flankiert.
"Beugung" ein Fragment der Länge k bezeichnet, das aus der Verlängerung eines Stammes mit einer Länge kleiner als die Länge eines k-mer der Länge k abgeleitet werden kann; und
"Stamm" ist ein Fragment eines k-mer der Länge k mit S definierten Basen, wobei S < k, und k-S ausgelassene (undefinierte) Basen sind; unter einem k-mer versteht man alle möglichen Teilzeichenketten der Länge k, die in einer Zeichenkette enthalten sind.

15. Computersystem mit einem Prozessor und einem Speicher, wobei der Speicher computerausführbare Befehle speichert, die, wenn sie von dem Prozessor ausgeführt werden, das System veranlassen, das Verfahren zur Identifizierung von Nukleinsäurevarianten zwischen zwei genomischen Zuständen durchzuführen, wie durch einen der Ansprüche 1 bis 10 definiert.

## Revendications

1. Procédé mis en œuvre par ordinateur pour l'identification de variants d'acide nucléique entre deux états génomiques comprenant les étapes consistant à :
A) Saisir 2 ensembles de lectures d'acide nucléique, qui sont des séquences récupérées d'un procédé de séquençage nucléotidique, dans lequel le premier ensemble de lectures correspond à des cellules représentant un premier état test, et le deuxième ensemble de lectures correspond à des cellules représentant un deuxième état témoin ;
B) Filtrer les lectures, dans lequel le filtrage comprend :
B1) Ne conserver que les lectures avec au moins un pourcentage X1 de leurs bases avec un score de qualité Phred supérieur à 20, X1 étant égal ou supérieur à 90 % ;
B2) Fractionner les lectures avec un nucléotide non défini, donner une séquence avant, et une séquence après le nucléotide non défini, ce dernier étant éliminé ; et
B3) Éliminer les lectures de séquence avec moins de X2 bases, dans lequel X2 va de 25 à 50 ;
C) Générer une structure de table de hachage comprenant :
C1) Générer un nombre de nouvelles lectures N-X2+1 pour chaque lecture de longueur de séquence N, dans lequel les nouvelles lectures N-X2+1 correspondent à tous les k-mers avec des nucléotides de longueur X2 ; et
C2) Construire une structure de table de hachage, qui comprend tous les k-mers générés à l'étape C1) et comprend en outre le nombre de fois que chaque k-mer est observé dans les deux ensembles de lectures correspondant aux premier et deuxième états.
D) Détecter des variants candidats dans la séquence entre le premier état et le deuxième état, dans lequel un k-mer de la structure de table de hachage est considéré comme un point de rupture candidat, qui représente un variant entre les premier et deuxième états, s'il remplit toutes les conditions suivantes :
D1) Au moins une inflexion basée sur une racine de k-mer doit avoir au moins X3 lectures avec la même variation entre les premier et deuxième états, X3 étant au moins 2 ;
D2) Le pourcentage de lectures du premier état dans les lectures du deuxième état n'est pas au-dessus d'un seuil X4, pour tenir compte d'une possible contamination de lectures d'état témoin avec des lectures d'état test, dans lequel X4 est d'au moins 5 %.
E) Regrouper et filtrer des lectures tests et témoins dérivées de tous les points de rupture candidats acceptés à l'étape D pour construire des blocs, en réalisant les étapes :
E1) Récupérer des lectures qui contiennent la racine d'au moins un k-mer qui représente le point de rupture candidat choisi à l'étape D) ;
E2) Les lectures de l'étape E1) avec au moins X5 variants de k-mer dans une fenêtre de X6 nucléotides sont considérées comme des lectures principales, dans lequel X5 est au moins 7 et X6 est au moins 10 ;
E3) Les lectures dont les k-mers partagent au moins une racine avec une lecture principale sont fusionnées pour donner un bloc ; et
E4) Si l'acide nucléique dont le variant est identifié est un ADN double brin, alors les variants avant et arrière sont pris en compte lors de la construction du bloc.
F) Aligner les blocs en prenant leurs premières lectures comme référence :
F1) Pour chaque lecture dans le bloc, prendre la racine de la lecture principale et trouver l'inflexion la plus longue ou l'inflexion partielle entre la lecture et la lecture principale.
F2) Positionner successivement chaque lecture de sorte que son inflexion correspondante ou inflexion partielle soit alignée sur la lecture principale ;
dans lequel :
« point de rupture candidat » désigne la position du nucléotide candidat où la séquence change, qui flanque immédiatement un variant de séquence.
« inflexion » désigne un fragment de longueur k qui peut être dérivé de l'extension d'une tige d'une longueur inférieure à la longueur d'un k-mer de longueur k ; et
« tige » désigne un fragment d'un k-mer de longueur k avec S bases définies, où S < k, et k-S bases omises (non définies) ; un k-mer étant entendu comme toutes les sous-chaînes possibles de longueur k qui sont contenues dans une chaîne.

2. Procédé mis en oeuvre par ordinateur selon la revendication 1, comprenant en outre l'étape suivante F2 :
F3) Obtenir des scores du premier état, des scores du deuxième état et des scores de similarité globale de chaque position dans le bloc en mesurant un rapport du nucléotide le plus fréquent dans cette position par rapport au nombre total de nucléotides.

3. Procédé mis en œuvre par ordinateur selon l'une quelconque des revendications 1 à 2, comprenant en outre l'étape consistant à :
G) Cataloguer et annoter des blocs selon ce qui suit :
G1) Si les blocs entre les premier et deuxième états ne diffèrent que dans un nucléotide substitué, le variant est catalogué comme contenant un variant de nucléotide unique et le variant de nucléotide unique est annoté ;
G2) Si les blocs entre les premier et deuxième états diffèrent dans plus d'un nucléotide mais que toute la différence de séquence est contenue dans le bloc, le variant est catalogué comme un petit variant structurel, et le petit variant structurel est annoté ; et
G3) Si les blocs entre les premier et deuxième états diffèrent dans plus d'un nucléotide et que toute la différence de séquence n'est pas contenue dans le bloc, le variant est catalogué comme un grand variant structurel, et les limites de tous les grands variants structurels sont étendues en récupérant des blocs chevauchant au moins X2 nucléotides dans un processus itératif qui se termine lorsque la séquence étendue atteint 200 nucléotides ou lorsqu'un chemin ambigu est trouvé.

4. Procédé mis en œuvre par ordinateur selon l'une quelconque des revendications 1 à 3, comprenant en outre l'étape consistant à :
H) Filtrer des blocs, selon ce qui suit :
H1) Le pourcentage de lectures du deuxième état dans les lectures du premier état n'est pas supérieur à un seuil X7, pour tenir compte d'une possible contamination de lectures d'état test avec des lectures d'état témoin, dans lequel X7 est d'au moins 20 % ;

5. Procédé mis en œuvre par ordinateur selon l'une quelconque des revendications 1 à 4, comprenant en outre éventuellement le mappage de blocs du deuxième état, et ensuite le mappage de blocs du premier état, sur un génome de référence.

6. Procédé mis en œuvre par ordinateur selon l'une quelconque des revendications 1 à 5,
dans lequel X2 va de 25 à 40.

7. Procédé mis en œuvre par ordinateur selon l'une quelconque des revendications 1 à 6, dans lequel X3 est égal ou supérieur à 3.

8. Procédé mis en œuvre par ordinateur selon l'une quelconque des revendications 1 à 7, dans lequel le seuil X4 est de 5 %.

9. Procédé mis en œuvre par ordinateur selon l'une quelconque des revendications 1 à 8, dans lequel le seuil X5 va de 10 à 15 et le seuil X6 va de 12 à 20.

10. Procédé mis en œuvre par ordinateur selon l'une quelconque des revendications 1 à 9, dans lequel le premier ensemble de lectures correspond à des cellules pathologiques d'un patient et le deuxième ensemble de lectures correspond à des cellules non pathologiques du même patient.

11. Produit de programme informatique comprenant des instructions de programme pour amener un système informatique à effectuer le procédé d'identification de variants d'acide nucléique entre deux états génomiques tel que défini dans l'une quelconque des revendications 1 à 10.

12. Produit de programme informatique selon la revendication 11 incorporé sur un support de stockage.

13. Produit de programme informatique selon la revendication 11 réalisé sur un signal porteur.

14. Système d'identification de variants d'acide nucléique entre deux états génomiques comprenant les étapes consistant à :
A) Des moyens informatiques/électroniques pour la saisie de 2 ensembles de lectures d'acide nucléique, qui sont des séquences récupérées d'un procédé de séquençage nucléotidique, dans lequel le premier ensemble de lectures correspond à des cellules représentant un premier état test, et le deuxième ensemble de lectures correspond à des cellules représentant un deuxième état témoin ;
B) Des moyens informatiques/électroniques pour le filtrage des lectures, dans lequel le filtrage comprend :
B1) Ne conserver que les lectures avec au moins un pourcentage X1 de leurs bases avec un score de qualité Phred supérieur à 20, X1 étant égal ou supérieur à 90 % ;
B2) Fractionner les lectures avec un nucléotide non défini, donner une séquence avant, et une séquence après le nucléotide non défini, ce dernier étant éliminé ; et
B3) Éliminer les lectures de séquence avec moins de X2 bases, dans lequel X2 va de 25 à 50 ;
C) Des moyens informatiques/électroniques pour générer une structure de table de hachage comprenant :
C1) Générer un nombre de nouvelles lectures N-X2+1 pour chaque lecture de longueur de séquence N, dans lequel les nouvelles lectures N-X2+1 correspondent à tous les k-mers avec des nucléotides de longueur X2 ; et
C2) Construire une structure de table de hachage, qui comprend tous les k-mers générés à l'étape C1) et comprend en outre le nombre de fois que chaque k-mer est observé dans les deux ensembles de lectures correspondant aux premier et deuxième états.
D) Des moyens informatiques/électroniques pour détecter des variants dans la séquence entre le premier état et le deuxième état, dans lequel un k-mer de la structure de table de hachage est considéré comme un point de rupture candidat, qui représente un variant entre les premier et deuxième états, s'il remplit toutes les conditions suivantes :
D1) Au moins une inflexion basée sur une racine de k-mer doit avoir au moins X3 lectures avec la même variation entre les premier et deuxième états, X3 étant au moins 2 ;
D2) Le pourcentage de lectures du premier état dans les lectures du deuxième état n'est pas supérieur à un seuil X4, pour tenir compte d'une possible contamination de lectures d'état témoin avec des lectures d'état test, dans lequel X4 est d'au moins 5 %
E) Des moyens informatiques/électroniques pour le regroupement et le filtrage de lectures test et témoin dérivées de tous les points de rupture candidats acceptés à l'étape D pour construire des blocs, en réalisant les étapes :
E1) Récupérer des lectures qui contiennent la racine d'au moins un k-mer qui représente le point de rupture candidat choisi à l'étape D) ;
E2) Les lectures de l'étape E1) avec au moins X5 variants de k-mer dans une fenêtre de X6 nucléotides sont considérées comme des lectures principales, dans lequel X5 est au moins 7 et X6 est au moins 10 ;
E3) Les lectures dont les k-mers partagent au moins une racine avec une lecture principale sont fusionnées pour donner un bloc ; et
E4) Si l'acide nucléique dont le variant est identifié est un ADN double brin, alors les variants avant et arrière sont pris en compte lors de la construction du bloc.
F) Des moyens informatiques/électroniques pour l'alignement de blocs en considérant leurs lectures principales comme une référence :
F1) Pour chaque lecture dans le bloc, prendre la racine de la lecture principale et trouver l'inflexion la plus longue ou l'inflexion partielle entre la lecture et la lecture principale.
F2) Positionner successivement chaque lecture de sorte que son inflexion correspondante ou inflexion partielle soit alignée sur la lecture principale,
dans lequel :
« point de rupture candidat » désigne la position du nucléotide candidat où la séquence change, qui flanque immédiatement un variant de séquence.
« inflexion » désigne un fragment de longueur k qui peut être dérivé de l'extension d'une tige d'une longueur inférieure à la longueur d'un k-mer de longueur k ; et
« tige » désigne un fragment d'un k-mer de longueur k avec S bases définies, où S < k, et k-S bases omises (non définies) ; un k-mer étant entendu comme toutes les sous-chaînes possibles de longueur k qui sont contenues dans une chaîne.

15. Système informatique comprenant un processeur et une mémoire, dans lequel la mémoire stocke des instructions exécutables par ordinateur qui, lorsqu'il est exécuté par le processeur, amène le système à effectuer le procédé d'identification de variants d'acide nucléique entre deux états génomiques tel que défini par l'une quelconque des revendications 1 à 10.
